# EUROPEAN PATENT SPECIFICATION

(11) **EP 4 312 905 B1**
(45) Date of publication and mention of the grant of the patent: **22.04.2026**
(21) Application number: 22776683.9
(22) Date of filing: 24.03.2022
(51) Int. Cl.: A61F 5/01

(54) **UNLOADING KNEE BRACE APPARATUS WITH CONFORMING AND DISTRACTING HINGE**
ENTLADENDE KNIESTÜTZVORRICHTUNG MIT KONFORMEM UND DISTRAKTIONSSCHARNIER
APPAREIL D'ATTELLE DE GENOU DE DÉCHARGEMENT DOTÉ D'UNE CHARNIÈRE DE CONFORMATION ET DE DISTRACTION

(30) Priority: 24.03.2021 US 202117211635; 24.03.2021 US 202117211590; 21.03.2022 US 202217700479
(43) Date of publication of application: 07.02.2024
(73) Proprietor: Icarus Medical, LLC, Charlottesville, VA 22902 (US)
(72) Inventor: JOHNSON, David, T., Charlottesville, VA 22903 (US); ECKERSLEY, Evan, Charlottesville, VA 22903 (US); SCIRE, Ben, Hopkinton, MA 01748 (US); HUBBARD, Emily, Purcellville, VA 20132 (US); WOO, Isaiah, Williamsburg, VA 23188 (US); FARILL, Collin, Charlottesville, VA 22902 (US); KITCHIN, Carter, Charlottesville, VA 22902 (US); FENG, Xue, Silver Spring, MD 20906 (US); RUSHTON, Charles, Dallas, TX 75214 (US); MILLER, Phil, Charlottesville, VA 22911 (US); MELLO, Tommy, Charlottesville, VA 22903 (US); JONES, Donald, Edward, Jupiter, FL 33477 (US); MOORE, Mason, Crozet, VA 22932 (US)
(74) Representative: Forresters IP LLP
(86) International application number: PCT/US2022/021822
(87) International publication number: WO 2022/204445

(56) References cited:
- US-A1- 2005 177 082
- US-A1- 2008 200 856
- US-A1- 2015 119 777
- US-A1- 2015 173 927
- US-A1- 2017 071 775
- US-A1- 2021 030 577
- US-B1- 6 527 733
- US-B1- 7 608 051

## Description

### FIELD OF THE INVENTION

The present invention disclosed herein relates generally to orthosis knee braces and methods of making them to relieve pain and discomfort by unloading the joint by redistributing the weight on the knee joint to other parts of the body and/or providing assistance in extension of the joint

### BACKGROUND OF THE INVENTION

Osteoarthritis (OA) is a degenerative joint disease characterized by chronic inflammation, breakdown, and eventual loss of the joint cartilage, causing deterioration of the underlying bone. The patellofemoral compartment, in particular, is one of the most frequent points of knee pain experienced by those with OA. While unloading braces have been used as inexpensive therapeutic solutions for knee OA, they have been overwhelmingly ineffective in preventing and reducing joint pain. The various embodiments of the present invention aim to design a knee brace that unloads the knee joint and reduces pain on a knee joint impaired by OA. The orthoses described herein are, among other things, focused on the transfer for forces away from an injured region of the knee, or unloading weight from the anterior region of the knee for patients suffering from patellofemoral pain (PFP) or patellofemoral arthritis.

Arthritis is currently the most common cause of disability among adults in the United States. More than one hundred different rheumatic conditions fall under arthritis, the most common of which is osteoarthritis (OA), a degenerative joint disease marked by a chronic deterioration of joint cartilage and the underlying bone. OA is one of the most common joint disorders in the United States, and the number of those afflicted is only projected to increase in the midst of an aging population and increasing levels of obesity. Twenty-seven million adults in the U.S. alone are affected by the disease. As the most typical type of arthritis, the disorder has commonly affected the knee, and the patellofemoral (PF) compartment within the knee joint in particular has been one of the most frequent points of knee pain in the outpatient setting. The PF compartment performs a key role in daily movement and activity, enabling mobility over a large range of motion through flexion, extension, and rotation of its associated components. One of the most non-invasive and widely accepted methods for prevention of further deterioration of the articular cartilage within the knee joint is by using a knee brace. The joint itself, including its underlying cartilage, can only support a certain amount of force before the cartilage begins to wear away, and unloading knee braces decrease the amount of force on the joint.

According to the American Academy of Orthopedic Surgeons and the U.S. Center for Disease Control and Prevention, nearly half of Americans develop symptoms due to knee OA by the age of 85, and the incidence rate for PF pain syndrome has been reported to be approximately 22 adults for every 1000 adults per year. In addition, up to 10 percent of the U.S. population suffers from pain and loss of function from patella arthritis and cartilage wear. The high prevalence of these injuries suggests that the condition affects a significantly large portion of the adult population and will have a growing impact on healthcare systems in the future. On average, total knee arthroplasty, or knee replacement surgery, costs between $10,000 and $30,000, and over 600,000 surgeries are performed each year. Other surgical procedures such as articular cartilage restoration, osteotomy, and uni-compartmental knee replacement, as well as corticosteroid and hyaluronic acid injections to reduce inflammation and absorb shock, respectively, are also very expensive. Thus, preventative treatments that reduce the amount of stress, pressure, and invasive procedures on the knee are necessary for improving the quality of life for patients and for reducing potential medical costs.

In addition, robust braces enable those with severe joint injuries to remain active when joint replacement is not appropriate. It is estimated that 27 million adults in the U.S. are suffering from osteoarthritis, and 454,652 patients with severe joint injuries and arthritis received knee replacement surgeries in 2004. Currently, nonpharmacological approaches, such as physical therapy, and pharmacological methods are primarily used to treat knee OA. When these are proven to be ineffective, the treatment method culminates to surgery, and drawbacks involve internal joint bleeding, bone healing failure, nerve or tissue damage, and infection. Thus, the development of a knee brace that significantly unloads force on afflicted joints, prevents pain and disability, and does not require many other treatments in conjunction is necessary to address the challenges associated with establishing a purely non-pharmacological, orthotic approach to treating knee OA. The main aim of the various embodiments of the present disclosure is to develop a knee brace that significantly unloads force from the patellofemoral compartment of a knee joint afflicted with osteoarthritis in order to relieve pain and disability.

Other conditions such as knee flexion contracture (KFC), quadriceps weakness, and neurological conditions that impact proper muscle and joint function, are also lacking in treatment options. A brace that can transfer forces away from the knee joint and quadriceps has promise to uniquely benefit patients who may suffer from these conditions. Transferring forces away from the joint may also allow the patient to delay or prevent surgical procedures such as a joint replacement.

### OA Knee Braces

Osteoarthritic knee braces primarily comprise a rigid, or semi-rigid, frame with an upper frame member called an "upper cuff" situated across the anterior thigh, and a "lower cuff" across the anterior or posterior tibia; and, straps are on the opposing side of the cuffs to secure the frame onto the user's leg. The upper and lower cuffs are connected by a rotary hinge assembly that pivots through a user's normal range of motion, or less depending on the injury.

In OA, the disease process includes degradative enzymes that erode the articular cartridge, leading to bone-on-bone contact, which is the primary source of the user's knee pain. OA knee braces classified as "unloading" braces pull the femur and tibia apart so that there is not bone-on-bone contact when the user is load bearing, such as walking, standing, exercising, etc. This is accomplished by the brace lifting the femur, and/or pulling down the tibia, or otherwise keeping the femur and tibia condyles from making direct contact through the actions of the upper and lower cuffs locking the femur and tibia in positions relative to the other.

Unloading knee braces may also comprise hinge assemblies that exert a force in the medial to lateral direction to push the knee joint inward, thus separating the femur and tibia condyles. For example, there may be one hinge assembly in the brace, such as for a brace to treat OA in the left medial compartment with a hinge assembly on the medial side of the knee joint; or hinge assemblies on both sides. The hinge assembly may comprise a component (e.g.an inflatable pad) that pushes the knee joint laterally, e.g. inward and/or apart, to unload forces on the medial side of the knee, and thus reduce the user's pain.

The hinges in unloading knee braces may also comprise components similar to a built-in breaking system where the user experiences an increase in tension as the knee is bent to prevent the user's knee from collapsing while bending. The hinge assembly and cuffs engage in a majority of the work that the leg muscles would otherwise do to stabilize the knee joint through its entire range of motion.

More recently, a number of OA knee braces have been marketed to consumers who wish to maintain an active life-style in spite of their medical condition. OA knee braces are now available that comprise hinge assemblies with the ability to exert forces to assist the user in movement, otherwise known as *"swing assistance"* or *"knee extension assistance".* The hinge's exerted restoring forces can be counter to the user's original direction of movement, such as propelling the user's knee from a flexed to an extended position after the user has bent down. The hinge assemblies primarily comprise springs and/or elastic members (tensioning elements) that store potential energy when the user is bending their leg, such as crouching down, during which the elastic members are stretched, or the springs members are compressed or stretched. The restoring force generated from the compression or stretching is used to assist the user when they move to extend their leg.

What is needed within the OA knee brace industry, though, is a knee brace that effectively both unloads the user's weight off the knee joint while dampening downward forces and generating restorative resistance forces that provide stability and support to weakened muscles. It would also be beneficial if the knee brace provides knee extension assistance to the active user. There is also a need for an improved mechanism of unloading that does not require pushing the knee inward, but instead relies upon a well-fitting rigid or semi-rigid frame and straps, and/or hinge assemblies that are of an adjustable tension that can be activated by the user as needed, and of significantly higher tension levels than the prior art's to engage in the mechanical work that is normally done by the muscles of the knee while pulling the femoral and tibia condyles apart.

The current invention provides a method and related software for the determination of measurements and instructions for the manufacture of medical devices based on a 3D (e.g., LIDAR) scan of a body part, limb, or joint. Furthermore, methods of interpolating accurate measurement data using specific algorithms, optionally developed through machine learning applications, are described.

Because of the required uniqueness of a custom orthotic device depending on the user, currently existing custom-fit medical technology requires significant time and precision to measure, design, and manufacture an effective product. Devices used currently, including calipers, measuring tapes, and casting techniques not only require significant time but also a high degree of skill, introducing opportunity for error if a professional is unavailable or untrained. It also limits the accessibility of such measurement solutions and increases cost overall by requiring a high degree of skill for accurate measurement determination.

In diagnostic cases (e.g., diagnosis of hydrocephalus in children) error in measurement can have chronic or even life-threatening consequences. For example, diagnosing microcephaly requires a practitioner to measure the head circumference of newborn, or recently born babies. A practitioner will then compare this measurement to a general population standard matrix, which comprises similar measurements submitted locally, nationally, historically. To read this measurement, a head circumference measure is obtained with a flexible non-stretchable measuring tape. Given the practitioner subjectivity of physical measurement tools, this process introduces levels of human error that would not be present using scanning technology and the inventive technique described herein. Similarly, human error can occur in goniometer readings, in the context of range of motion monitoring in patients. If a practitioner is unavailable, or untrained, these measurements can vary, or be generalized.

An alternate scanning and processing method could capture a number of data points representing the limb's motion in real time, and more accurately monitor the range of motion of a patient. Additionally, the invention described herein shortens the time required to create custom medical technology for the alleviation of pain, structural support, or realignment of joints, bones, or limbs. Numerous medical measurements lack accuracy and/or are prone to error because they are difficult to physically measure with calipers or with 2-dimensional images, such as ensuring that parts of the body are aligned during a joint replacement procedure. By reducing user error involved with traditional measurement tools and providing additional data by which a better fit can be informed, the invention can also increase the precision of the fit to improve comfort and wearability while reducing sliding and addressing major limitations of other braces that reduce user compliance, adoption, and overall efficacy. Through capture and processing of data including 3D data, patient data, radiographic data, and biometric data, the invention can achieve a higher degree of customization with the aim of joint restoration, pain relief, or fulfilling other unique user needs.

The high degree of precision yielded by the scan, ease of use, and high degree of reproducibility cannot be produced by existing measurement technology and methods, as such methods do not provide sufficient safeguards against human error. A "smart" measurement tool that can identify quality of data, interpolate or extrapolate additional data, or transform data to improve data quality to improve the manufacturing outcome can be yielded through the described disclosure. For example, one consideration in the fit of a custom knee brace is the body mass index ("BMI") of the patient and correlating amount of adipose tissue within the thigh. A knee brace could have improved fit based on degree of adipose tissue present and need of compression for adherence to the thigh, and may consider the type and severity of an injury so that an appropriate corrective force may be applied, and for example, optimize the location of an axis of rotation based on data that represents the envelope of joint motion, which may be collected based on a series of scans or images, a video, or a 3D video capture, as described herein. The number and types of measurements, as well as the precision required can be optimized, selected or calibrated based on the required application, clinical indication, manufacturing technique and desired output. Additionally, the more advanced measurements can be gathered that are beyond the capability of standard tools to properly capture, for example an accurate Q-angle of the knee or a precise volume of a limb.

Some other applications may be useful in record-keeping, diagnosing, or making other medical decisions, such as detecting body volume and composition changes, including swelling, which may enable the early detection of peripheral arterial disease (PAD) in patients with diabetes for example, that may lead to limb amputation. Another application is to spatially map the relative distances of objects, at an instant or with time, and compare these with nominal anthropomorphic values to assess whether a problem exists and possibly whether a physical change should be made. An example of this application is assessing a potential airway characteristic or obstruction that may be indicative of increasing the probability of sleep apnea, or another breathing disorder. The wide range of applications of this innovation is not limited to humans, and can be used for sizing limbs, bodies, or body parts for animals in a similar manner. This technology incorporates 3D scans and a range of possible biometric data, and can therefore be used to help predict not only corrective forces for bracing outside the body, but also devices that could be implanted within the body. For example, the shape of a joint replacement or a component that can be added to a joint or limb can be modified to improve function. The addition of a component, body, or structure that can alter the system of forces in a joint or limb to either reduce pain or improve function or performance is possible with the method described herein. Surface information combined with biometric insights would enable developing an appropriately sized and positioned component, body, or structure to achieve a desired purpose. A similar approach can be taken for non-biological applications, for example, specifications can be generated for a structure composed of layered materials with different strength properties when combined with information from a surface scan.

### Description of Related Art

Within the relevant industry, custom fit medical devices currently are able to form fit the patient using a three dimensional digital representation of the body or a casting of said body part. This technique is well known in the field of dental orthotics where it is common to make a silicone mold of the patient's teeth using an upper and lower tray, make a plaster casting of the mold, and scan the cast to generate a 3D representation of the teeth. In a similar manner, a 3D scan of a human or animal limb can be used as the basis to fabricate a custom orthotic or brace. However, the currently-existing processes exists only for individual customers and cannot be successfully scaled to large groups, which would allow for a reduction of cost and time, a more available product, and more consistent, high quality products. Therefore, there is a need for the innovations described herein.

Additionally, the design of devices made from methods that are based on 3D scan data - which typically only consider the external surface of the body part - does not account, for example, for internal deformities and / or biomechanical needs. Thus, those devices may fail to provide corrective forces to achieve an optimized or improved biomechanical state. Likewise, a surface scan used to capture the shape of the limb or body part for fitment does not take into account patient specific needs such as the location of skin ulcers, areas of tenderness, bone spurs, etc., that would necessitate the alteration of the orthotic to be worn comfortably. It is a limitation that the medical devices currently produced from 3D scans are focused heavily on form-fitting and space constraints.

Further, the current state of technology lacks an autonomous process for generating a computer-aided design (CAD) for a custom-fit medical device. Accordingly, there exists a technological need for an autonomous process taking into account multiple design elements above and beyond conforming to the existing body part surface - including but not limited to orthotic strength, comfort, pain relief, form fitting, patient features including joint geometry, degree of OA in each joint, amount of adipose tissue (e.g., extrapolated by BMI), that is truly custom to the patient's shape as well as their indications, envelope of motion, axis of rotation, and gait pattern.

Thus, there remains a general need for enhancing the digital model characteristics of an orthotic or device derived from body surface models to achieve specific function by using relevant physiological and / or biometric data.

US 7,608,051 discloses an osteoarthritis knee orthosis, which comprises a polycentric hinge positioned on one side of the patient's knee and a unicentric hinge is positioned on a second side of the knee.

### SUMMARY OF THE INVENTION

The present invention has been described with reference to particular embodiments having various features. It will be apparent to those skilled in the art that various modifications and variations can be made in the practice of the present invention without departing from the scope of the invention. One skilled in the art will recognize that these features may be used singularly or in any combination based on the requirements and specifications of a given application or design. Embodiments comprising various features may also consist of or consist essentially of those various features. Other embodiments of the invention will be apparent to those skilled in the art from consideration of the specification and practice of the invention.

The present invention provides a joint brace as claimed.

Various embodiments of the present disclosure comprise a novel type of unloading knee brace that has been designed to reduce the amount of pain that patients experience as a result of PFP or knee joint OA. The knee braces and hinge assemblies disclosed herein work by using one or a combination of the following mechanisms:
a. generating a force or forces opposing the bending or contracting/flexion of the knee joint,
b. applying a force or forces to the sides of the leg that result in unloading one or more compartments of the knee,
c. hinges that distract forces from one or both sides of the knee in the tibiofemoral compartments, and/or
d. hinges that are curved that conform to the shape of the users knee.
The braces described herein are suitable for a knee joint, an elbow joint, an ankle joint, a shoulder joint, a hip joint, or a wrist joint; while application of this technology for the knee joint will be presented here in detail, one of ordinary skill in the art could readily apply this disclosure and teachings herein to an elbow, hip, shoulder, ankle or a wrist brace. The brace effectively unloads a significant amount of force within the knee by using a low- to high-tension resistance mechanism described herein, and by distributing the force to other areas of the body. This results in reduced contact pressure in areas of the knee afflicted with OA, and therefore less pain. Other braces are described that have different mechanisms of unloading or distracting pressure in joints as well.

Some embodiments described herein allow the user to: quickly (e.g., in aspect from about 1-5 seconds, for example) engage and disengage tension in each joint mechanism as needed, including in aspects while the brace is being worn; adjust the amount of tension, including while the brace is being worn and without the need for a medical professional's assistance; allow for tension to increase with increasing degrees of flexion; and limit the range of extension and flexion in the joint. The device is particularly suitable for people afflicted with patellofemoral osteoarthritis (OA), cartilage damage, meniscus damage, knee stability issues, and other types of knee conditions for which pain intensifies during the bending or contracting of the knee, and for patients who lack the strength (e.g., quadriceps weakness) to extend their knees either during exercise or simple life functions, such as standing from a seated position. The various embodiments of the brace and hinge assemblies disclosed herein provide enhanced support for stabilizing the knee joint, and they can enhance the user's physical performance by providing extension assistance. Another version of the brace may have the tensioning elements oriented such that the brace resists extension and assists with flexion. The tensioning mechanism may be used with or without the device. This type of device can treat knee flexion contracture, which may be used to help patients recovering from joint replacement surgery improve range of motion.

The various embodiments of the brace and hinge assemblies disclosed herein may also be applied to other orthotics designed to treat other human joint, such as elbows, shoulders, ankles, wrists, and hips, wherein a support in one part of the joint is operatively connected to another part of the joint via a variety of the tensioning mechanisms described below that may alter the amount of force between parts of the joint. For example, a shoulder brace may apply a posteriorly directed force through the shoulder to unload the anterior direction, or other direction(s), to alleviate pain. In this example, part of the shoulder brace will attach to the injured part of the body and will anchor to another part of the body, such as the shoulder opposite to the injured shoulder. Rigid and semi-rigid parts may be used in conjunction with the tensioning mechanism to create the desired force environment for the joint.

### Unloading Brace Vertical Support

The various embodiments of the present disclosure comprise a knee brace that effectively unloads the user's weight off the knee joint via a rigid or semi-rigid vertical support that, in aspects, partially or completely connects to the user's femur and tibia, and with a pivoting hinge assembly connecting an upper and lower portion of the vertical support. In some embodiments, the posterior side of the vertical support comprises one to three straps on the upper and on the lower portion, or other mechanisms to connect the device to the knee, or elbow, or ankle, by way of example, which may be oriented in a variety of ways relative to the vertical support.

It is noted that the vertical support of the present disclosure may also be used with a wide variety of types of hinge assemblies previously known in the art for use by knee patients in order to effectively unload weight from the knee j oint. One such assembly may be the combination of rigid and semi-rigid materials that enable the brace to be connected to or contained within an elastic sleeve or support that partially or fully encompasses the joint.

The various embodiments of the knee brace comprise a vertical support with an upper frame and a lower frame that are connected via a hinge assembly on one side (for a medial or lateral brace), or via two hinge assemblies (for a full brace). Furthermore, in embodiments, the vertical support comprises an arcuate, curved, semi-circular rigid or semi-rigid unit situated above and below the knee, and connected via a geared or ungeared, pivoting hinge assembly, in examples. The upper and lower frames may further comprise at least one strap or other connection mechanism to secure the brace to the user's leg; and the upper portion may have straps and/or a material for supporting the back of the thigh to effectively distribute force away from the knee. The upper and lower portions may also be secured with a hook and loop type material, or a clip-type fastener or similar method.

The brace can be one-sided or bilateral (as in a right and left, or medial and lateral support), the determination of which is based on whether the knee is injured medially or laterally, or in the femoral compartment, which is approximately central. The tensioned brace hinge assembly should be proximal to the injured part of the knee. A user benefiting from a high-tension brace would ideally use a brace with both lateral and medial side supports to generate torque on both sides.

The amount of torque can be modified by the strength and number of elastic materials, and the amount of torque may vary on each side to address the user's specific OA condition. The brace frames disclosed herein are capable of targeting damage to the patellofemoral compartment; but other types of knee injuries and medical conditions may benefit from only a side support vertical member and/or one hinge assembly.

The brace can also accommodate a patient who experiences more symptoms of OA in one compartment of the knee than the others, by applying a force on the opposing side of the unicompartmental OA present in the patient's knee. This can be achieved by a variety of methods known to the art; for example, the condylar pad on one side of the joint may be stiffer or thicker than the condylar pad on the other side of the joint. The use of shims that can be connected to the side plate or hinge capsule and adjusted based on the degree of varus/valgus present in the user's knee may also apply.

Another application of the brace may involve using the same tensioning mechanisms taught herein to assist flexion instead of opposing flexion. This could be accomplished by changing the location of the tensioning element such that it is located on the posterior side of the hinge assembly and encourages flexion of the joint by maintaining tension in the tensioning element. This application of the device would be useful for those recovering from an injury or undergoing physical therapy, as an indication of knee health after recovery is the range of motion (flexion) the knee can achieve as swelling decreases in the joint.

The brace can be partially or fully automatically designed by software that generates a design based on a 3D scan consisting of cloud point data, accompanied by information on the user such as the severity of their injury, information on the misalignment based on radiographic measurements, measurements extrapolated from 3D scans, and other user biometric data such as height, weight, and age, or any combination of this information. The model developed can also use this information to develop implantable devices within the joint to improve joint function. These may also be used to serve as a substrate for cartilage growth on the implant or to shape how cartilage grows in the joint in a desired way. The implant may also be placed to assist cartilage growth in another area that is near or related to the implant. Artificial intelligence or machine learning can be used with or without finite element analysis to determine the shape of an effective or assistive implant, and AI, machine learning, and FEA, or combinations thereof may consider all of the input data above to improve overall body mechanics. This approach may be applied to more than just the knee, but also the ankle and hip, and how these joints relate to each other to improve body function. This can also be applied to the shoulder, elbow, wrist, back, and neck joints, or combinations thereof.

**Brace Materials:** In embodiments, the vertical support is made from rigid and/or semi-rigid plastic, metal, other lightweight materials, such as carbon fiber or another suitable material that are mostly inelastic yet flexible, and thus distribute weight-load knee forces. 3D printing with common thermoplastics are ideal materials for fabricating the brace described herein.

Because the knee braces are subjected to high tension or high torque from the hinge assembly, tight, form-fitting contact with the body is preferred. The brace may further comprise light padding lining the upper and lower portions, and/or the straps or other connection mechanisms. In aspects, the fit and material composition are designed to provide a coefficient of friction between the brace and a user's leg so as to increase adhesion to the user's leg, and thus facilitate the transfer of weight-load forces off the knee joint, while remaining comfortable to wear. The upper and lower portions and/or straps or other connection mechanisms can be contained or built within an elastic sleeve to reduce the friction coefficient at the body/brace interface. The fabrication method combined with using strong and lightweight materials will facilitate this design feature. Furthermore, the brace can be made from common materials, such as braided tensioning elements, where applicable, and may therefore be less expensive and more accessible to users that may not normally be able to afford a performance brace. 3D-printed versions of the brace frame may have padding that is 3D printed continuously or separately attached. 3D printed padding comprises a compressible matrix that conforms to the body and provides cushion.

In aspects, the brace frame, or vertical support, comprises: an upper rigid, or semi-rigid, frame, sized to fit a user's femur adjacent to and above a user's knee joint, and a lower rigid, or semi-rigid, frame, sized to fit a user's tibia adjacent to and below the user's knee joint.

*Size:* The knee brace can be custom made for the user based on one or more of: size, weight, level of physical activity of the user; weight and flexibility of the brace; etc. Or it can be sold over-the-counter based on size (for example, small, medium or large), and/or by level of tension (low/medium/high). Or, the brace may be custom made to fit a particular user using digital imaging. In a preferred embodiment, the brace is form-fitting to the knee joint, lower femur, and top tibia, in order to redistribute the load off of the knee joint, when the device is being used to unload forces. The brace may conform to digital images or a three-dimensional scan and this fitting process may be automated or partially automated. Software can orient the leg in the proper direction and scale the leg and brace properly.

### Hinge Assemblies

In embodiments, the present invention comprises at least one tensioning element (e.g., a tensioning element, an elastic band, or a spring) of low, moderate, or high tension, two intermeshed, teethed gears rotating in unison as the user flexes and extends the knee joint, and a method of controlling the degree of extension and flexion the joint can achieve while the user is wearing the brace. Other embodiments do not require teethed gears but instead are toothless with curved adjacent parts that glide on each other and are made from a low-friction material, in aspects.

Hinge assemblies can be used with the unloading brace vertical support disclosed herein; and/or with other knee braces or joint braces known in the art.

In the hinge assemblies disclosed herein, the amount of tension for unloading can be adjusted by, for example: adding more tensioning elements of the same or of different levels of tension; adding more tensioning elements of the same or of different levels of diameter; and/or by substituting tensioning elements with different elastic properties (e.g., stiffer bands or springs to create more tension; and/or by moving a hinge component to fix one end and/or the center of a tensioning element to prevent it from further extension, thus increasing the tension in the element (e.g., see embodiments 2-4 of the hinge assembly, *infra*)). The hinge assembly may include smoothed sections to prevent damage to the tensioning element(s), and to allow for drawing-stretching-extending the tensioning element over the hinge, whether or not the tensioning element(s) come into contact with the rotating gears.

When more than one tensioning element is used, the tensioning elements may be located, in certain aspects, adjacent longitudinally in the anterior side of the hinge assembly, and/or the posterior side, such as in parallel, or one atop the other, such as in series. Another design feature, in aspects, is that a plurality of tensioning elements improve safety of the brace by providing a backup support in the unlikely event that a band breaks or detaches.

If a tensioning element is to be used, in embodiments, hinges with bands as large as 19,05 mm (¾ inch) and as little as 3.175 mm (⅛ inch) in diameter are envisioned, and larger and smaller bands could be used in the same brace.

Tensioning elements having different levels of tension comprise materials, in examples, such as: real rubber, braided synthetic rubber cords, exotic elastic or other elastic materials. Braided bands offer protection to the elastic material, and other bands can use thin protective sheaths or a wet or dry lubricant to allow for smooth drawing over the hinge, in aspects. Bands that are 3D-printed with many individual elastic strands oriented in the direction of tension will make a preferred tensioning element.

An additional hinge assembly may be envisioned that is comparative to the gliding and rolling of the knee joint. This version may involve a slot that allows for the gliding and rolling motion of the knee wherein the slot is a pin in the upper and or lower frame. A tensioning element may be anchored between the upper and lower frames to slow or to impede the forward movement of the knee joint, in a way that best matches the natural movement of the knee.

### Single-upright mechanism:

**Extension-Flexion Stops:** In addition to a controllable tensioning mechanism as described in embodiments 1-5, for example, extension-flexion stops may be used to prevent users from hyperextending or hyperflexing joints that may already be prone to injury, as well as simply limiting the degree of flexion or extension. In disclosed hinge assemblies herein, the brace hinge may comprise various methods of controlling the degree of extension and flexion of the joint. In one inventive embodiment, a slot that is radially oriented to the pivoting point of the hinge can be cut out of the hinge or created during fabrication of the joint. In other words, the hinge may comprise a slot, for example a slot that is radially-oriented to the pivoting point of the hinge. The degree of flexion and extension that the angle between the two frames of the brace can achieve during an articulated joint movement can be controlled by placing premade inserts at chosen locations in the radially oriented slot. The premade inserts can be fabricated with the material strength and shape to withstand articulated and intense joint movements without fracturing, bending, or slipping out of the slot. Either the side plates or hinge capsules will help secure the inserts into the hinge, without restricting smooth motion of the hinges/intermeshed teethed gears. This slot and inserts at various points in the brace allow for user customization of degree of flexion and extension allowed by the device at the hinge point.

Another variation of the extension-flexion stops is through a fabricating process, wherein inserts that may be placed in between the anterior and/or posterior of the upper and lower frame hinges can be used to control the degrees of flexion and extension in the joint at, for example, the hinge point. For example, the insert may be placed on the anterior side of the frame hinge between the gears in order to limit the degree of extension of the joint; alternatively, the inserts may be placed on the posterior side of the hinge to limit the degree of flexion of the joint.

The geometry of the teethed gears and hinge(s) may also be altered upon fabrication of the hinge grouping in order to limit the degrees of extension and flexion the joint can perform during use of the brace or a limit on the degrees of flexion and extension allowed by a hinge point on the device. For example, the geometry of one gear can be designed such that it does not fit within the opposing intermeshing gear at a certain degree of flexion or extension. The teethed gears/hinge preferably comprise a durable material that can resist the tendency for movement during extension or flexion of the joint.

Tubes: In another embodiment, the hinge assemblies comprise a tube or tubes through the geared components in the brace, and/or the brace frame, and comprise a tube or tubes within the support structure. The tubes may be integrated partially or completely within the frame, or may be external to the frame. The tube(s) in the brace components may be balanced to offer sufficient strength while minimizing the bulk and weight of the component. Materials can be chosen to allow for smaller or larger sized brace components. The tubes may be located anywhere within the frame of the brace and may orient the bands in a plurality of ways depending on a user's need, treatment, preference, comfort, injury, performance requirement, etc.

Tensioning elements can be located at different positions within the brace-above and/or below the hinge, and the hinge can draw lace or wire over the hinge causing the tensioning elements to elongate. The tension in the system would be modulated in a similar way, and the tension adjustment mechanism can be placed at various locations on the brace.

Another feature includes using tensioning elements that have a distinct ending point that limits the degree of flexion based on the length of the tensioning element and the length of the component, by limiting the amount of band drawn over a section of the hinge that acts as a cam, which generates a mechanical advantage as it draws the tensioning element(s) apart from its anchored ends. For example, in embodiment one, the bands are fixed at both ends; in embodiment two and four, *infra,* for example, the band(s) are fixed at the distal end only and tension is adjustable at the proximal end; and in embodiment three, *infra,* the band(s) are fixed at both ends, but are adjustable for tension. The shape of the cam can be modified to increase or decrease the elongation of on the tensioning element and therefore affect the torque generated.

The tension, or counter-force, in the hinge assembly may be adjusted by: increasing the number of tensioning elements to increase the tension, and/or by using tensioning elements of more stiffness for a higher tension. In an embodiment, the knee brace is manufactured for a specific tension (low, medium, and high). In another embodiment (e.g., the second through fifth hinge embodiment, *infra*), the tension is adjustable by deactivating a hinge mechanism to allow the tensioning element(s) to stretch, or by activating the mechanism to block the tensioning element from stretching on one or both ends, thus increasing the tension in the band(s).

In another embodiment, comprising multiple bands, the elements can be mixed or combined with different strengths and sizes based on the user's preferences or needs, and the different elements can be engaged at different degrees of flexion. For example, one band could be engaged from 5- 20 degrees of flexion, by way of example only, at which point another band would engage to increase the resistance.

The bands can be secured through a number of methods, including the use of clamps or pins or anchors through the tensioning element or through which the tensioning element may be engaged, and hole(s) in the brace may comprise components to prevent the band ends from slipping out of hole(s) while the brace is under tension. Other band geometries can be used, such as circular bands that hook into the top and bottom components of the brace.

The distal and proximal hinge are preferentially fabricated as a continuous material with the vertical supports, or alternatively are secured to the brace frame by bolts, rivets, pins, screws or another similar attachment mechanism. A brace support may be of plastic or carbon fiber and could be shaped to include the tensioning element supports and the gearing mechanism. An unloading brace can be made through any a combination of 3D printing, injection molding, water-jetting, casting, extruding, pultruding, or other similar ways. This brace can use multiple injection-molded components that connect together and house tubes, tensioning elements, and/or wires on, or partially or completely within, the components. These components may be connected to metal frame parts that generally shape around the leg or other limb. This version of the brace may be an alternate version of the 3D-printed version as a lower-cost or higher-volume production alternative.

The hinge components on the lateral and/or medial side of the knee can be spaced snugly to keep a narrow profile. If multiple elastic materials are drawn across the hinge, they can be oriented vertically or horizontally to the desired dimensions and/or tension of the brace. The components can be symmetric or shaped to contour the leg.

The hinge that connects the top and bottom components of the brace can, in aspects, be a U-shaped joint or another component that will offer lateral stability to the brace. These can be threaded or designed in a way to minimize the size and profile, such as using E-clips (circlips) or pressing the components in place.

### Additional Applications

The hinge and tensioning assemblies described herein may be applied to other human joints, including but not limited to the ankle, shoulder, hip, elbow, and wrist joints. These embodiments of the present invention may include a support of one part of the joint being operatively connected to a support of another part of the same joint. This connection may comprise a tensioning element, which may or may not be adjustable, so that the brace may apply force in a direction favorable for rehabilitation or support of a joint.

For example, in an embodiment, an ankle brace may comprise an ankle cuff and a lower portion that connects to a region or regions of the foot. The ankle cuff may be connected to the lower portion of the ankle brace by one or more materials and/or adjustable tensioning elements that will apply force to desirable locations of the ankle and foot in order to provide the ankle with more support.

In another embodiment, the brace may comprise a portion that can be secured on one end to the hip of a user and to the leg of the user of the other end. By connecting these two ends of the brace with a tensioning element, the ball and socket joint of the hip may be adjusted to better align the femur and pelvis in a way that is physically preferable for the patient.

Any additional embodiments of this brace, as they are applied to other joints, may employ a variety of optionally adjustable tensioning elements, such as combinations of tensioning elements in series or parallel, and the strength of the tensioning elements may be adjusted depending on the type of joint and treatment needed per user. These additional embodiments may also employ the adjustable tensioning mechanisms, *infra,* in order to allow for dynamic use of the brace.

### Tension Adjustment and Engage/Disengage Features

Another feature of the brace design taught herein is that in embodiments two through five, *infra,* the user can either fully or partially disengage the tension mechanism. The tension engagement-disengagement feature allows the user to increase the tension in the hinge assembly to provide more stability and off-loading of their weight from their knee, such as when climbing stairs, and then to turn off the mechanism or decrease tension when it is no longer needed, such as at the top of the stairs, so that the user can more easily walk or jog with a fuller range of motion. The current invention allows for this adjustment in real-time or near-real-time and while the user is wearing the brace.

### Embodiment 1- Fixed Tension

*Hinge Assembly 1:* In a first embodiment, the pivoting hinge assembly comprises two opposing, facing subunits, with a proximal (top) and distal (bottom) short end, and an anterior (front) and posterior (rear) side. Each subunit houses one gear that intermeshes with an opposing gear during articulated joint movement, e.g. a proximal and distal gear; at least one tensioning element extending between the subunits on the anterior side of the gears and fixedly connected on the band's ends to the posterior side of the subunits; and a connector on the medial and lateral side pinning the subunits together while allowing the gears to rotate. Tension may vary in the hinge depending on the strength of the tensioning element provided in the hinge assembly; this may be decided at the time of fabrication of the brace. Alternatively, the hinge may freely pivot without teethed gears.

### Hinge Assembly- Embodiment 2- Adjustable Tension- via Handle and Slider

*Hinge Assembly 2:* Various embodiments of the present disclosure further comprise a second embodiment of a hinge assembly for use in a brace as disclosed herein, or other knee brace for treating a medical condition that requires unloading of a joint. The hinge assembly of embodiment 2 is similar to embodiment 1, but with the addition of a handle or knob attached to a mechanism that enables the user to adjust the tension on one end of the tensioning element(s) in real-time or near-real-time and in aspects while the user is wearing the brace by pulling the handle or knob one direction, thereby increasing tension, and then decreasing or releasing tension by moving in another direction.

In one embodiment, the tensioning element(s) proximal end is attached to a slide member that moves vertically (e.g., proximally-distally or distally-proximally) to, in aspects, pull the band taut to increase its tension. For example, when a user moves a handle or other mechanism that is located on the outside portion of the hinge, above the knee (or in aspects below or beside the knee), it moves backward-posteriorly. This handle movement forces a connecting slide member to move up-proximally, thus stretching the proximal end of the tensioning element(s). Thus, in an embodiment, the user can increase the stability and/or stiffness and/or tension of the brace/hinge/tensioning element by moving the hinge handle backwards, then moving it forward-anteriorly to release or decrease the tension and make the brace more flexible, which may comprise a fuller range of motion. In other aspects, the handle may slide front to back, back to front, or diagonally. In aspects, the user can increase stability and/or stiffness and/or tension of the brace/hinge/tensioning element by moving the hinge handle forwards, upwards, downwards, sideways, or diagonally, and then releasing or decreasing tension by moving the handle in an opposite or different direction.

### Hinge Assembly- Embodiment 3- Adjustable Tension via Ratchet-Pawl

*Hinge Assembly 3:* In another embodiment, each subunit houses one gear that intermeshes with an opposing gear during articulated joint movement, e.g. a proximal and distal gear; and at least one tensioning element extending between the subunits on a side of the gears (or over or under the gears) and fixedly connected on the tensioning element's ends to the posterior side of the subunits. In aspects, a core bracket member completely or partially covers the tensioning element between the subunits' open space to protect the element(s), and to pin the gears together while continuing to allow them to move relative to one another.

This embodiment may further comprise a rotatable or linear ratchet-pawl member on the upper and/or lower frame of the brace to vary tension in the band or bands. The user can rotate the knob or slide a lever to different positions to pull the tensioning element(s) tighter while reducing their effective length; this may be accomplished by winding a part of the tensioning element, for example a wire, around a coil as the member is rotated. For example, rotating the ratchet-pawl members clockwise increases the tension in the hinge assembly, making it less flexible, off-loading more of the user's weight from the knee joint, and providing more stability. The user can then release the ratchet-pawl members by pulling up or pushing down on a knob or a deactivation lever that is co-located with the member (or turning the knob in an opposite or different direction or rotation); and/or, in turn, the user would then be able to rotate the knob in a second, opposite direction to relieve tension in the tensioning element stretched between the gears.

### Hinge Assembly- Embodiment 4- Adjustable Tension- Spooled Wire

*Hinge Assembly 4:* The various embodiments of the present disclosure may further comprise another embodiment of a hinge assembly for use in a knee brace as disclosed herein, or other brace for treating a medical condition that requires unloading of the joint. The embodiment comprises one or more strands of tensioning elements with the elements' respective ends fixed in the distal subunit. The element(s) endpoint on the proximal end is pulled on by a wire that encircles it.

In this embodiment, a rotatable knob is connected to a spool of wire, for example, that pulls on the proximal end of the tensioning element as the user rotates the knob (if the knob is on the proximal portion; if knob is on the distal portion, the distal end is pulled). In aspects, the knob is rotatable to fixed positions so that the user is able to adjust the tension in the tensioning element to a desired level, and release the tension by rotating the knob in a different or opposite direction. In aspects, more turns or longer turns on the knob will result in higher tension in the tensioning element, and more off-loading of forces on the user's knee joint.

### Hinge Assembly- Embodiment 5- Wire-linked Bands with Adjustable Tension

*Hinge Assembly 5:* The various embodiments of the present disclosure further comprise another embodiment of a hinge assembly for use in a knee brace as disclosed herein, or other brace for treating a medical condition that requires unloading of a joint. This embodiment comprises one or more tensioning elements housed completely or partially within the frame of the brace in both the proximal and distal frame portions. The one or more tensioning elements are further connected to each other by a wire that stretches over the gear assembly, and one or both bands are connected to an adjustable tensioning mechanisms using another wire(s).

In this embodiment, equal tension should be applied to the one or more bands in the hinge assembly, and tension is generated within the frame of the brace to generate resistance to flexion. The adjustable tension mechanisms of embodiments, 2, 3, and 4, for example, as explained *supra,* are connected to at least one of the bands either directly or indirectly.

### Method of Use- Embodiments 1-5

In various embodiments of the present disclosure, the amount of weight unloading (or resistance or tension generated in the brace) can readily be tailored to a user based on their size, weight, injury, therapeutic needs, and/or desired athletic performance. Braces as described herein are capable of being lightweight, robust, of a narrow side profile, and well-fitting to users. Unlike braces in the prior art, those disclosed herein can be narrow and lightweight so as to be worn under clothing, which is usually not possible for athletic performance braces. For these reasons, the brace can be ideal for a range of injury types and severity, as well as a way to enhance athletic performance.

The various embodiments of the knee brace of the present disclosure can be used, by way of non-limiting examples: prophylactically to prevent injury; to reduce joint pain (e.g., during normal activities, physical exercise, or athletic competition); to rehabilitate existing injuries; postoperatively (high tension braces to immobilize the joint to a comfortable level); as extension assist devices for medical conditions such as osteoarthritis, with some stability support for proper knee alignment through the range of motion; to enhance athletic performance (e.g., by applying force as a knee joint extends to, for example, add explosiveness as an athlete jumps or starts running); and/or to prolong the life of a natural knee afflicted with osteoarthritis or other knee injury, or to prolong the life of a prosthetic joint, possibly in order to delay, prevent, or avoid knee surgery.

Likewise, the knee brace and/or hinge assemblies disclosed herein are able to: reduce the weight, forces, and/or pressure on a knee joint when a user is load bearing on their legs, such as standing. And/or, the knee brace and hinge assemblies are able to provide knee extension assistance when walking, bending, moving from sitting to standing, exercising, etc.; therefore, the user has to exert less physical effort to move their knee between flexion and extension.

In an embodiment, the method of use for reducing load bearing on the knee joint comprises the steps of: attaching a knee brace of, for example, one of the embodiments listed above to a user's knee, comprising laying the inside surface of the brace vertical support comprising the upper and lower portions against a user's leg; and closing the brace straps or other way of connecting the brace to the user, such as multiple straps around the user's femur and multiple straps around the user's tibia; and, load bearing on the user's knee joint, wherein the load and/or pressure on the knee joint is reduced to the extent that the user experiences a reduction in pain or an improvement in movement as compared to load bearing without the knee brace.

A method of use further or alternatively comprises extension assistance, comprising the steps of the following when the user flexes a knee joint: stretching and generating a counter or restoring force at the hinge tensioning element to propel the hinge back from a bent, flexed position to a straight, extended position; wherein the brace reduces the amount of force required to be exerted by the user's leg and knee and associated muscles to return the brace hinge (and knee joint) to an extended position from a bent position; and wherein the load and/or pressure on the user's knee joint is reduced to the extent that the user experiences a reduction in pain or improved movement as compared to flexing and extending the user's knee without a knee brace.

In yet another embodiment, a method of use comprises: having a user activate a hinge mechanism to pull one end (or both ends) of the tensioning element(s) more taut to increase tension and stability in the hinge assembly and knee brace, and then to deactivate the mechanism when it is no longer needed (or decrease tension). Various embodiments of the hinge mechanism comprise: a handle or engaging piece attached to a sliding lever, wherein moving the handle backwards (or forwards, upwards, downwards, or diagonally) causes the sliding lever to move in manner to pull one end (or both ends) of the tensioning element(s) taut (e.g., see second embodiment, *supra*); a rotatable or linear ratchet-pawl mechanism on one or both ends of the hinge (or above or below or beside the hinge) that a user can move clockwise or counterclockwise (or up or down) to impinge the tensioning element(s) and increase tension therein, then release (see, e.g., third embodiment); and a rotatable knob connected to an internally housed spool of, in aspects, rigid line or wire that is attached to a folded tensioning element, wherein turning the knob pulls on the tensioning element to increase the band's tension, and rotating the knob in the opposite direction releases or decreases the tension (see, e.g., fourth embodiment).

### Methods of Generating Tension

Tension may be generated in a hinge by drawing a tensioning element, which may be an elastic, semi-rigid, or rigid component or components, across a hinge or lever arm that results in the elongation of the tensioning element. The tension generated as a function of degrees of flexion or per degree of flexion may vary throughout the range of flexion to generate different force profiles and resulting device performance. This may be achieved by variation in hinge geometry such as including a cam on the hinge or the pathway of the band or tensioning element, or gears of different radii. The geometries of cams or gears of variable radii may be tailored to achieve a desirable unloading profile depending on the user's needs. For example, a cam or gear may have an exaggerated bulge as opposed to a flat shape, which will generate an increasing amount of tension per degree of flexion as the user proceeds through a range of motion because the tensioning element or wire connected to the tensioning element will travel further over the cam. The force profile may be, by way of example only, linear, logarithmic, or exponential. Cams or gears of different geometries may be built into the brace or rapidly engaged, disengaged, or interchanged using levers or switches to achieve different force profiles and performance as needed.

Overall tension or the adjustment of the force unloaded per degree of flexion could be changed by limiting the movement of elastic material or anchoring at various fractions of the length, therefore limiting the region of elongation. The radius of the gear, thickness of the band, length of the band, multiple of bands, and other attributes of the brace can be adapted to the user's knees.

Bands or tensioning elements may comprise smaller individual bands or bundles that may be activated in parallel to achieve varying degrees of tension and unloading.

The activation of one or more tensioning elements may or may not be accomplished electronically via piezoelectric sensors or other electronic signals activated manually or automatically.

This system of generating tension can be applied to other joints of the body that may or may not include geared or ungeared hinges. For example, flexion and extension in the wrist may be assisted in a similar manner by connecting an upper and lower component above and below the wrist joint that are operatively connected using a tensioning element that may or may not be adjusted with a rotational dial such as a BOA or other mechanism.

Tension may be applied across various mechanical joints within the device, such as ball- and-socket, condyloid, gliding, and saddle joints. It may be applied in one or multiple axes, and can be engaged or disengaged as a whole, or in one axis depending on user need. The joints alone or in combination with the tensioning mechanism may limit or allow movement in one or multiple axes to reproduce, augment or limit the natural movement of the anatomical joint to which the device is applied.

### Generating tension across a single upright hinge

A single upright knee brace may include a tensioning system. A single upright knee brace may provide valgus or varus unloading support by utilizing material flexibility, rigidity, and strength characteristics to apply forces in the desired location(s). For example, a brace designed to treat varus to relieve medial compartment osteoarthritis may apply a lateral force in the center of the brace. The curvature of the frame away from the leg will apply a medial force above and below the user's knee, resulting in a corrective or distractive force that will reduce pressure in the medial compartment. Alternatively, a valgus brace may pull the joint laterally to unload the medial compartment while applying a lateral force above and below the joint. These forces may occur in conjunction with a force that pushes or pulls the joint apart, allowing for less contact pressure between joint components, such as the femur and tibia in the tibiofemoral joint.

The amount of force and support may be based on user inputs such as self-reported pain levels and measured or estimated inputs such as Q-angle and radiographic information.

In embodiments, the single upright brace frame is designed to translate force optimally without the bulk and weight of a double upright brace. For example, in aspects, the proximal and distal posterior support is required and an anterior support is required above and below the knee. In aspects, the brace can be worn on the medial or lateral side of the leg. In aspects, the brace can treat varus or valgus from either the inside or outside of the leg. For the unloading variant, the tensioning element may be drawn over the hinge and tensioned using one or more elastic bands and a tension-locking mechanism such as a BOA dial or other tension-adjusting mechanism that may be placed on the upper or lower portion of the frame. The brace may be contoured around the leg and patella to prevent or minimize rotation around the leg and migration down the leg. An alternative version of this brace has a fixed tensioning element. A strapping system may be incorporated into the brace frame that provides or supports the varus or valgus correction, prevents rotation around the leg, prevents migration down the leg, and eliminates undesired torques that the joint may be subjected to.

A variant of the knee brace may use the same brace and tensioning system to generate tension or resistance in the opposite direction, as in it resists extension. The device may be used to assist users by increasing range of motion.

### Bolt action or lever mechanism as an adjustable tensioning system

An alternative method of tensioning a band or multiple bands is with a system of one or more levers. A lever or levers may be pulled in one direction to increase tension and hooked onto a latch to maintain tension. The lever may be unlatched by pulling in one direction and moved by pulling in the other direction. The lever may be located on the upper or lower member of the knee brace and may be on one or both sides of the knee brace. The lever is operatively connected to the tensioning element. The lever may involve lace or wire to connect to one or more tensioning elements and one or more pulleys may be involved.

### Cam mechanism as an adjustable tensioning system

The tension in the brace may also be adapted to individual users by adding a cam-like feature wherein the tensioning element is drawn over a part causing the band or lace or wire to travel a greater distance as a result of the cam than without it. The result of the implementation of this mechanism may be an increased amount of tension per degree of flexion and may be tailored to individual users. The cam system may be adjusted on inputs from radiographic information and assessed need for correction and support. The cam modifications may be fully or partially automated and incorporated into the design process of the brace, or modified after the brace has been fabricated. The cams themselves may be adjustable and are interchangeable, in aspects.

### Internalized band as an adjustable tensioning system

An alternative system involves a mechanism where tensioning elements are integrated within the brace frame rather than drawn over the hinge. These bands may occur as one or more and may be found in either or both the upper and lower members. The brace frame may involve a tensioning system that controls the amount of torque around the hinge.

### Method of joint distraction to generate space in between the joint

In a knee brace as described herein, distraction can be accomplished between the tibia and femur in a number of ways. In aspects, one way is in a geared or ungeared hinge, the radius of the gear can vary with degrees of flexion. One example is as the degree of flexion increases, the distance between joint centers may also increase, thereby providing a separation force across the joint. A user may be assessed to determine the optimal variation in the gear radii. In embodiments, for a double upright knee brace, the gear radius on one side of the brace can differ from the gear radius on the other side. This method of joint distraction is also applicable for a single upright knee brace. This mechanism may or may not include a slot design that allows for limited direction of travel based on gear radius. The slot controls the direction of the distraction. A center cap encases the hinge and contains slots to support the hinge mechanism. These slots can be oriented in an optimal manner for the user's needs and may be linear or curvilinear.

The distraction hinge may or may not be associated with a tensioning system. When the brace does not include a tensioning system, tension may be applied to the center pin through the hinge to maintain close gear contact. A mechanism of restricting motion of the center pin through the slot such as a leaf spring or a clip or clips may or may not be implemented.

The vertical member may be elongated to increase distraction. In embodiments, this can be accomplished in a number of ways such as providing a telescoping vertical member where the distal end of a member and the proximal end of a member are increased or decreased in distance based on the user's needs. Another method according to the present invention is the use of a spiral rotating knob that can push the distal and proximal members apart. The result is an increased distance between the connection points of the upper portion and lower portion of the knee brace to the user's leg. This can also be accomplished electronically by a motor tensioning the system. The telescoping mechanisms can be located on the upper or lower or both portions of the knee brace. The system may rely on one or more pulleys.

### Pivoting external loops as a mechanism to secure straps or semi-rigid supports

A pivoting external loop or pivoting D-ring is included wherein the D-ring component includes a slot for passage or connection to a strap or support which may be comprised of Velcro or another material. The pivoting member involves a partially approximately round component that allows for the position of the strap relative to the frame to pivot. The pivoting member either straps into or is embodied within the brace frame. The component may or may not be 3D printed and may or may not be printed within the existing structure. Alternatively, the D-ring may be continuously adhered directly to the brace frame and may be cast, injection molded, or 3D printed. The range of motion or positioning of individual D-rings can be customized or altered based on the user's needs or the position on the brace frame.

### Method of detailed characterization of knee for brace design

A user's knees may be characterized by qualitative assessment through various activities such as squatting or knee extension. Input to the brace design may be extracted from radiographs, x-rays, MRIs, 3D scans, or data collected from sensors. Additional inputs may include patient-reported pain score, Q-angle, measurement of adipose tissue, and radiographic information. Radiographic information may or may not be used to estimate the firm and soft tissue in the user's knees. Aspects of this process may be automated.

The radius of the gear, thickness of the tensioning element(s), length of the tensioning element(s), multiple of tensioning element(s), number of tensioning element(s), and other attributes of the brace can be adapted to the user's knees. The process of assessing the patient as a method of creating inputs for the desired brace design can be partially or fully automated. For example, the Q angle may be estimated by a knee scan or x-ray, and a patient may report a certain level of pain in one compartment of the knee, such as the medial tibiofemoral compartment. The design of the brace may be automated to modify the amount of force applied to the opposing joint compartment (lateral) and other points on the leg to alleviate pressure in the injured joint compartment. The device may engage or disengage and alter tension in response to EMG or other biometric data related to the user's movement and support or limit movement in a desired manner.

From inputs such as biometric or radiographic data, the nature of the user's injury can be characterized and elements such as Q-angle correction, amount of unloading force based on degree of flexion, torque profile for medial and lateral sides, and tension levels for medial and lateral sides may be determined. The nature of the injury for the knee's envelope of motion may be understood. This may be modeled based on a scan based on the range of motion of the joint.

### Adjusting joint geometry/gait via tensioning

The user's injury will be analyzed with data including but not limited to MRI scans, x-rays, qualitative information such as self-reported pain and region of joint, and biometric data such as BMI and Q-angle. The patient may be recorded and gait modeled as well. This information can be used through manual or automated processes to restore and improve joint geometry to reduce pain or enhance performance. The brace may automatically or partially automatically adjust to the user's needs.

Joint rotation and/or joint alignment may be influenced by varying tension across the hinge. Tension may be applied to one or both sides of the brace. If tension is applied on both sides of the brace, the tension may be equal or dissimilar to generate the desired torque profile for each side. The torque profile is designed based on the needs of the user's joint to restore or improve joint alignment and function. The size and geometry of the brace frame may be modified to flex the appropriate amount. Material choice such as plastic, metal, carbon fiber, or a combination thereof may be used to achieve the desired flexibility to support the goal of improving joint function. The amount of flexibility of the brace frame will be tailored to the needs of the user for fit and to assist with generating an unloading force. For example, a brace with a bowed frame with the center of the bow placed at the center of the side of the knee condyle will generate an unloading force in the compartment opposite of the side where the bow is contacting the knee joint.

The user's gait may be influenced to a healthier gait by generating an appropriate amount of tension. The information described in the previous section and sections herein can improve gait, alter walking pattern to reduce pain, increase stability, and reduce long-term wear on the joint.

### Method of securing wire or lace to tensioning element

The tensioning element within a brace may be either 3D printed based on an elastic polymer or elastic material such as rubber, or may be cast or injection molded from a similar material. The wire or lace or cable may be secured to the tensioning element by looping through a series of holes and knotting or tying off the lace or wire. Alternatively, the lace or wire may be knotted or placed within a hardened material such as an epoxy that is then integrated or bonded or attached to the elastic material. The region where the wire or lace attaches to the tensioning element may use a metal component that may be crimped or compressed to secure the wire or lace within the tensioning element or elements. A part to increase surface area may be tethered or attached to the wire or lace and inserted within the tensioning element to prevent the band or wire or lace from detaching. The system may be secured further by coating with a glue or hardening compound such as an epoxy to minimize the risk of detachment. The opposite end of the tensioning element may be anchored in position due to a change in geometry that would prevent the tensioning element from moving or dislocating. Alternatively, this part may be pinned, glued, pinched, or screwed in place to prevent dislocation.

### Method of securing knee orthosis to ankle orthosis

The braces described herein may involve connection to an ankle foot orthosis wherein the tensioning system for the knee brace may additionally be connected to the ankle orthosis. Alternatively, the position support or tension in the ankle orthosis may be controlled by a separate tensioning system on the knee portion of the orthosis or ankle portion of the orthosis. The knee orthosis may be operatively connected to the ankle orthosis by a plastic, metal, carbon fiber, or other structural material. The geometry of the orthosis may be based on a 3D scan from the patient's leg and/or foot. Corrections to the gait and points of support and pressure may also be based on information from the 3D scan. In aspects, for unloading Knee Ankle Foot Orthoses (KAFOs), the amount of loading assistance will be pre-set in the brace according to the size and strength of the tensioning elements based on the user's needs. When tensioning elements and a tension locking mechanism are used, the tensioning elements may be drawn over the hinge or hinges, or attached to a wire or wires that are drawn over or through the hinge. The devices may interface mechanically or structurally to generate dynamic and synergistic forces throughout the lower limb through a range of motion or gait. For example, the tensioning elements of the knee brace and ankle orthosis may be mechanically integrated so that as the knee reaches about 60° of extension, the ankle orthosis support provides dorsiflexion support. Additionally, the tensioning elements may communicate remotely via Bluetooth, WiFi, or other signaling means to work synergistically to support or direct gait.

### Method of Making

The various embodiments of the present disclosure may use traditional manufacturing processes for knee braces, and/or 3D printing to produce prototypes or final versions of the components (such as the gears and/or subunits of the hinge assembly) to then be injection molded, extruded, pultruded, or may be entirely 3D modeled and/or printed, from parts to the entire brace. In an embodiment, the brace is sized to fit the user and can be form fitted to the user. Unique fabrication methods and materials make this form fitting brace possible. For example, two-dimensional or three-dimensional pictures, videos, or scans can be used to generate a model or a final product (or parts) that contours or fits the user's leg or other joint, and the properties of the material, in aspects, will have an amount of flexibility in the lateral direction, for example, and less flexibility in the direction of extension or extension depending on the purpose of the brace.

The fabrication technique of the braces herein allows the braces to include unexpected advantages not included in the prior art, including manufacturing and performance advantages. Therefore, an improved fitting brace that is higher functioning, safer, more effective, and more comfortable is possible by the invention taught herein. The fabrication methods and materials can also assist in keeping production costs lower than the prior art.

In addition to injection molding and 3D printing the frame of the brace, the brace may also be constructed entirely of a material that allows for it to be thermal molded around a specific patient's leg post-fabrication, or similarly, sections of the brace may be made of a material that can be thermal molded to produce a specific force on a patient's leg at a given location, for example providing varus/valgus support. Additionally, it may be desirable for the padding on the brace, whether it is 3D printed as an extension of the frame of the brace or separately adhered using another method, to be thermal moldable to a patient's leg. The benefit of this would be that the padding could possibly be switched out or modified (if it is not continuous with the brace) as the patient desires, without the need for refabrication of the brace. Pultrusion and extrusion techniques are also envisioned.

### Unloading and Torque

The knee brace vertical support of the present disclosure differs from the prior art, including in that it unloads a significant amount of force that is normally applied within the knee. The basis for patellofemoral pain is that a large amount of force is distributed over a small area. Injuries to this surface can result in severe pain and defects/injuries, and the cartilage surface can degrade, thus exposing bone and nerves in an accelerated time frame. The tension-generating, unloading mechanisms in the present disclosure's knee brace address distributing forces experienced in the knee to other body parts and dampening the impact that would be painful to a joint afflicted with osteoarthritis. The effect of action of the brace is equivalent to a significant reduction of weight by the user; the most fundamental treatment for sufferers of osteoarthritis is weight loss.

The amount of force unloaded in a knee brace of the present disclosure is characterized by its relative torque measured about the hinge (e.g., in units of Newton-meter ( inch-pounds) [in- lbs]), and the amount of weight unloaded or offset (in units of kilograms (pounds) [lbs]). For example, the general strength or tension of the knee brace of the present disclosure is generally broken down into three categories:

| | |
|---|---|
| Low: | below 3 lbs. unloaded |
| Medium: | range of 3-15 lbs. unloaded |
| High: | above 15 lbs. unloaded |

where 3lbs corresponds to 1.36 kg and 151bs corresponds to 6.80 kg.

The reduced force in an OA afflicted knee joint via use of the present brace and/or hinge assemblies allows for deeper flexion of the user's knee that would normally be prohibited due to pain. This deeper flexion engages the user's quadriceps to an extent that would normally be avoided by the user due to debilitating pain, thus facilitating a user gaining strength through exercise. Additionally, the resistance generated by the brace can strengthen supporting soft tissue during exercise, for example the hamstring can be strengthened via a brace vertical support and/or hinge assembly as disclosed herein that resists tension on the quadriceps.

### Use of Condyle Spacers

The knee brace described herein may include condyle pads that may or may not be increased or decreased in width depending on the severity of the varus or valgus alignment of the knee. Condyle spacers are used to shift the Q angle of the knee, or the angle of the femur relative to the tibia. A method of correlating the Q angle to the degree of varus and valgus has been developed, and this may automatically generate inputs into a digital model of the brace to be fabricated in order to sufficiently compensate for the medical condition.

**In** embodiments of the present invention, the condyle portions of the brace can be adjusted in a telescoping manner to increase or decrease pressure on one side of the joint. For example, a certified prosthetist orthotist may be required to evaluate the Q angle of a user's knee, and then assign a specific number of condyle spacers that should be inserted within the adjustable condyle hinge region of the brace. The condyle spacers may be inserted by removing the screws and caps of the condyle hinge and inserting the desired number of condyle spacers into the condyle region of the brace, and replacing the cap and screws after adjustment. This embodiment poses the advantage of being able to readjust or add to the width of the condyle region if a progressive treatment path is desired for a patient. Another variation involves sliding spacers of different sizes in a tongue-and-groove that may lock or snap into positon, and allow for rapid adjustment of the condyle spacing.

**In** additional embodiments of the present invention, a predetermined width of the condyle spacing region may be desired. In this embodiment, the width of the condyle region would not be expected to change throughout the course of treatment for the patient, and the width of the hinge would be determined during fabrication of the brace, such that the condyle hinge cap of the brace may have a thickness that is determined based on the desired correction of the Q angle of the knee.

### Use of Sensors and Motors

The knee brace described herein may have sensors in place that measure and monitor the position of the brace relative to either or both the leg and another part of the brace. This position data can provide velocity and acceleration data that are used as inputs to a processor or monitoring system for the brace. Velocity and acceleration may be measured by positioning sensors or other sensors. This data may provide the basis for adjustment by a motor system to either assist or support a joint by increasing or decreasing tension.

Sensors may also be used to measure and monitor the amount of tension present in the brace or joint assistive device, and the amount of unloading force applied at the joint, including a variable amount that changes as the joint is extended or flexed. The analog value of the tension present at the joint may be converted to a digital signal in a variety of ways, such that the user of the brace has knowledge of how much tension is present in the brace at any given time, or as a change in tension is recognized by the sensor.

The sensor(s) may be fabricated on or within the brace. The sensor(s) may output a digital or electronic signal, and they may connect to one or more LED lights that may indicate the information about the brace such as the amount of force or tension in the brace at any given moment in time. Additionally, the sensor(s) may be connected to one or more lights that light up different colors depending on the amount of force or tension in the brace; for example, the light may light up one color for maximum force and another color for a lighter amount of force.

The brace motor, sensor, and control processor system may also include a potentiometer, gear box of gearing system, and one or more servo arms or levers. The motor is operatively connected to the tensioning element through a system of gears or another method such as a screw, which can gather or release tension, based on inputs from sensors managed by a controller or processor.

The sensor(s) may also be connected to a screen on the brace that communicates information such as force generated within the brace, or weight unloaded by the brace, such as in a relevant unit value for the user. The sensor(s) may also be synced to an application on a smart device, such as a smartphone, tablet, or computer, that provides the user with feedback about the amount of force being applied by the brace, and/or the direction the joint is being overloaded in or the direction the joint is being flexed or extended. Data from these sensors may be logged and analyzed, used to identify patterns, and may be used as inputs to a controller that determines how motors should function in an assistive or supportive manner.

The sensors may also be connected to the tensioning mechanism; for example, using feedback from the sensors, the tensioning mechanism may loosen or tighten the tension in the tensioning elements based on the feedback it receives and a preset level of desired tension, as decided by the user. This would eliminate the need for the user to adjust the amount of tension present in the brace during use of the brace. In an embodiment, a user would set certain parameters and, based on feedback from the sensors as processed by a processor, the brace would be able to automatically adjust tension using motors, hydraulics, or microdrives, for example, or to alert the user to change resistance. In aspects, the sensors and related processor may be connected to a server or the internet, which may inform the processor of whether to adjust tension, or it may provide advice to the user about tension recommendations or other information related to treatment or use of the brace. Similarly, the sensors in adjunct with a processor may inform a doctor of the tension in the knee brace or other information from the brace and use of the brace so that a treating physician, for example, could diagnose the patient, monitor the patient, monitor the treatment, provide treatment options, warn the user of problems, adjust tension, determine when there is improper use of the brace, determine if an injury has occurred, monitor performance, etc. Thus, sensors used with a processor may be able to provide more automatic use and adjustment of the brace, including using software implemented predefined parameters to adjust tension or otherwise monitor and control use of the knee brace. The tension may also be adjusted electronically in the absence of sensors, where one button or input increases tension, and another button or input may decrease tension. This can be done with a toggle switch, rotatable knob, or touch button(s).

Electromyography (EMG) sensors may be used to activate a joint assistance mechanism to unload weight in the joint for which the device is applied. This may be done with or without other sensors and with or without motors. The degree of assistance may be modified and calibrated to the needs of the user.

The current invention provides for a system and method that, in aspects, comprises the capture of a 3D point-cloud data set and optionally incorporates additional data, clinical requirements, lifestyle objectives, or physiological data. It further comprises processing the data, selecting a reference point or feature within that 3D point-cloud data set, defining a series of critical points from the point-cloud data set with a relation to the selected reference point, and determining digital specifications, instructions, or diagnostic readings based on the clinical application. The process is capable of interpolating or extrapolating additional critical points to restore the 3D model or scan and meet necessary criteria for measurement determination.

In an initial stage of the process, in an aspect, data (such as a scan or model and its underlying data) representing the 3D digital object of one or more parts of the body is captured. In aspects, a digital 3D surface model or scan captures the following: a series of points generated by a 3D scan and a negative of a body part representing the surface morphology. (At times herein, this may also be referred to as a 3D object, 3D digital object, 3D digital model or scan, 3D digital scan or model, or digital representation.) The object or model or scan data may be generated by a digital scan using an application ("app") on a cell phone, tablet computer, computer, digital camera, or other digital device, and/or a compilation of two-dimensional data sets (e.g., photos taken from different angles). The 3D digital object or scan data represents a surface to which a brace or device may be relatively fit or contoured. The 3D digital object or scan data may be generated indirectly, for example by making a cast of the body part and then scanning the cast or a mold from the cast.

3D digital models or scans may be captured through a range of motion capture to record or measure the envelope of motion of a joint (e.g., the knee from full flexion through full extension).

Additional data may be collected and input to the system manually or automatically. Such data may include, for example, quantitative data from a prescribing doctor including Q angle of the knee, patient biometric and user data such as body mass index ("BMI"), and qualitative data such as degree of patient pain, location of pain, and pain during movement. Further data may be indirectly generated through pre-defined functional relationships to initial data inputs. For example, the degree of elasticity or mechanical impedance of the thigh, calf or other body part may be calculated from age or BMI based on previously defined functional relationships or algorithms. Another example is the determination of required ultimate strength, yield strength, mechanical advantage, or other specifications of a device based on the user's daily work or activity.

Radiographic, ultrasonic, or other quantitative clinical data may be included to instruct on device shape for corrective purposes, to restore a healthier joint geometry, or to unload a region of the joint.

When the 3D point-cloud data set is captured, it may be rendered within a computer-aided design software or other visualization software. An engineer or specialist may select one or more reference data points within the data set, for example the center of a patella, as a reference point. From the reference point, additional reference points may be selected or designated automatically based on their relation to the initial data set of the feature(s). For example, points within a specific distance or following a certain path from the initial data point may be used to define additional points for designation and assessment of the feature(s). Extreme points medial and lateral to the center of the knee could be selected in such a way, representing center points of the knee condyles. From the manually or automatically designated reference points, a series of critical points are designated. The critical points may be defined automatically to meet a minimum requirement for accuracy of measurement determination based on the target device manufacturing or diagnostic process specifications.

The critical point(s) comprise(s) a data set by which measurements can be determined directly, for example as a circumference of a path connecting the outermost critical points within the data set at a distance from the reference point. For example, by selecting the center of the patella of a leg scan, a set of critical points represented as a discrete set of data points within the scan would be defined, which may constitute a mesh. The path length of specific circumferences at the knee center, 15.24 cm (6") above the knee center, 15.24 cm (6") below the knee center can be directly generated.

In some instances, the 3D point-cloud data set yielded from the 3D scanning process is not sufficient enough to determine the measurements as required by the process or protocol specification. In such a case, additional critical points are interpolated or extrapolated from the existing data set. Several applications may occur based on specific algorithms, potentially determined from machine learning applications. Such interpolations or extrapolations of additional data points may comprise scan extension, scan repair, or calibration and manipulation of segments of the scan itself. For example, a flexed knee scan can be manipulated into an extended knee scan.

The user may be presented directly with measurements for use in the selection or manufacture of a properly fitting medical device. Alternatively, the measurements may be incorporated into instructions for the manual fabrication of the device.

The software may be fully integrated with a fabrication system (e.g., 3D printing software) to automatically enter a fabrication queue for manufacturing. The entire process from scan to print and even through assembly and packaging may be fully or partially automated based on specific application programming interfaces, in aspects, be completed within under an hour. In other embodiments, the disclosed invention may be applied for diagnostic applications.

### BRIEF DESCRIPTION OF THE DRAWINGS

The accompanying drawings illustrate certain aspects of some of the embodiments of the present invention, and should not be used to limit or define the invention. Together with the written description the drawings serve to explain certain principles of the invention.

A reference object is defined as one or more points, lines, curves, splines, axes, shapes, planes, cross-section, other two-dimensional objects, meshes, wireframes, other three-dimensional objects, and/or a combination of the preceding list.

A 3D point-cloud data set can comprise a set of data generated by a scanning (e.g. LIDAR) technology to yield a digital representation of an object.

A reference point can be a user or system selected point within the 3D point-cloud data set that represents a feature around which measurements can be determined, and it can be used to orient and calibrate additional critical points within the 3D point-cloud data set.

A critical point can be a data point within the 3D point-cloud data set that is defined relative to a reference point and has assigned spatial coordinates to yield an amount of information necessary to determine a set of measurements based on the specific application.

A mesh represents a set of critical points organized in a regular or irregular pattern.
FIG. 1 is an illustration of the anterior left view of the knee brace frame in the extended position.
FIG. 2 is an illustration of an exploded view of the anterior left view of the knee brace frame in the extended position.
FIG. 3 is an illustration of an anterior left perspective view of the knee brace in the extended position with no adjustable tensioning mechanism.
FIG. 4 is an illustration of an exploded view of the anterior left view of the knee brace with no adjustable tensioning mechanism.
FIG. 5 is an illustration of a left side view of the single upright embodiment of the knee brace with a combination of rigid and semirigid supports.
FIG. 6 is an illustration of an anterior left view of the knee brace embodiment with an internal adjustable tensioning mechanism.
FIG. 7 is an illustration of the anterior view of a knee brace embodiment with teethed gears comprised of variable radii that provides joint distraction.
FIG. 8 is an illustration of the exploded, posterior right view of the knee brace embodiment with teethed gears comprised of variable radii.
FIG. 9 is an illustration of the anterior view of a section of the knee brace embodiment with conforming gears and hinge caps and an adjustable tensioning mechanism.
FIG. 10 is an illustration of the anterior view of a section of the knee brace embodiment with conforming gears and hinge caps without an adjustable tensioning mechanism or tensioning element over the hinge assembly.
FIG. 11 is an illustration of the anterior left view of a knee brace frame as an embodiment with a cam mechanism as an adjustable tensioning system.
FIG. 12 is an illustration of a hinge section view of an embodiment of the knee brace frame with a cam mechanism as an adjustable tensioning system manufactured with 3D printing techniques.
FIG. 13 is an illustration of an exploded anterior left view of the knee brace frame with a slot system as a means of distraction.
FIG. 14 is an illustration of a left view of an embodiment of a knee brace in flexion with a slot system as a means of distraction.
FIG. 15 is an illustration of an exploded anterior left view of the knee brace frame with a slot system as a means of distraction with no adjustable tensioning system.
FIG. 16 depicts an exemplary form of a fully or partially automated process, with vertical integration of generating a medical device from data collection (e.g., scan) to fabrication.
FIG. 17 depicts an automated process according to the invention described herein with a rendered base file for a custom knee brace (1), its transformation to fit to a 3D digital object based on geometric constraints (2), and completed generation of a custom knee brace following addition of components and features, and quality control (3).

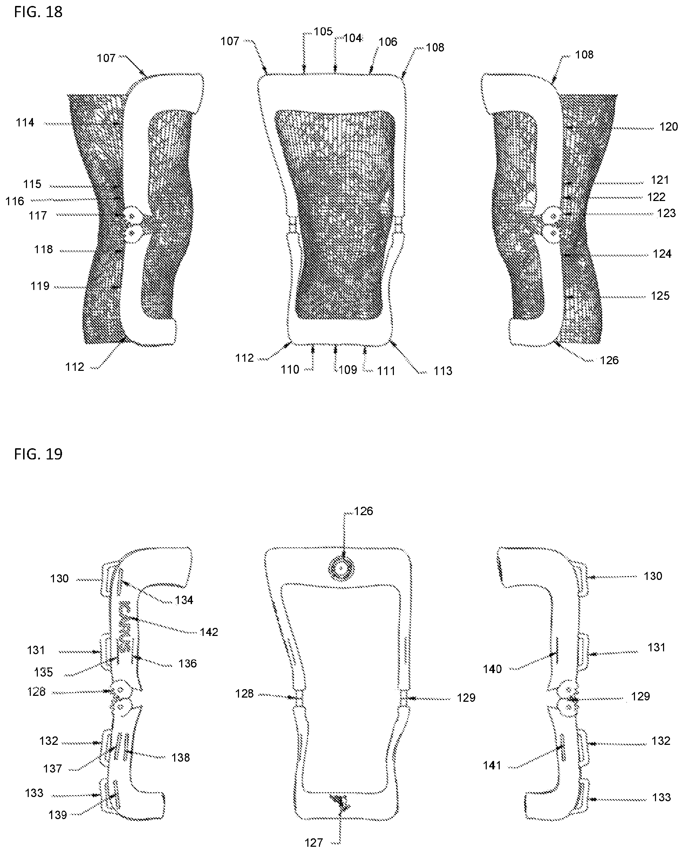

FIG. 18 depicts an example of what a computer aided design file could perform in stage 3 of Fig. 16. Numbered elements 104-125 point to a reference object. A combination of these bodies creates one or more medical devices.
FIG. 19 depicts an example of what a computer aided design file could perform in stage 4 of Fig.1. Element number 126 indicates a tensioning sub-system. Element number 127 indicates an engraving of the company logo. Element numbers 128-129 indicate a rotation system. Element numbers 130-133 indicate a strapping sub-system. Element numbers 134-141 indicate an additional strapping sub-system. Element number 142 indicates an engraving of the company name.
FIG. 20 depicts the high-level representation of stage 3 of Fig. 16. Element number 143 indicates a reference object. Element number 144 indicates input data. Element numbers 145-147 represent the axis coordinate system. Element numbers 148-150 represent the rotational freedom of a reference object. Depending on the reference object, the degrees of freedom may be limited--meaning that the reference object may not be able to follow the direction numbers 145-150.
FIG. 21 depicts an exemplary form of a fully or partially automated system that operates the fully or partially automated process depicted in Fig. 16 according to an embodiment of the current invention.
FIG. 22 depicts an exemplary form of a fully or partially automated process for the scanning of a limb to generate a 3D point-cloud data set and collection of additional biometric data, selection of the reference point(s), designation and spatial orientation of critical points, modification of critical points based on biometric data, and output of specifications or diagnostics, according to aspects of the present invention.
FIG. 23 depicts an exemplary representation of the process applied to a scan of a leg for the determination of specifications for fabrication of a knee orthosis, according to aspects of the present invention.
FIG. 24 depicts an exemplary scan requiring data interpolation or scan repair, and the functional data set that is yielded to collect measurement data, according to aspects of the present invention.
FIG. 25 depicts an alternate representation of the designation of reference and critical points using a cylindrical coordinate system, by way of example, according to aspects of the present invention.

### DETAILED DESCRIPTION OF EXEMPLARY EMBODIMENTS

As used herein, the term "proximal" is synonymous with top or upper, as in above the knee, or the side closest to the user's torso. Likewise, the term "distal" is synonymous with bottom or lower, as in below the knee, or the side furthest from the user's torso.

As used herein, the term "anterior" refers to the front of the knee and/or brace, and "posterior" the back. As seen in the figures when the hinge is oriented up-down, anterior is upward, and posterior is downward.

Throughout the following detailed description the same reference numbers refer to the same elements in all of the figures.

### Knee Brace

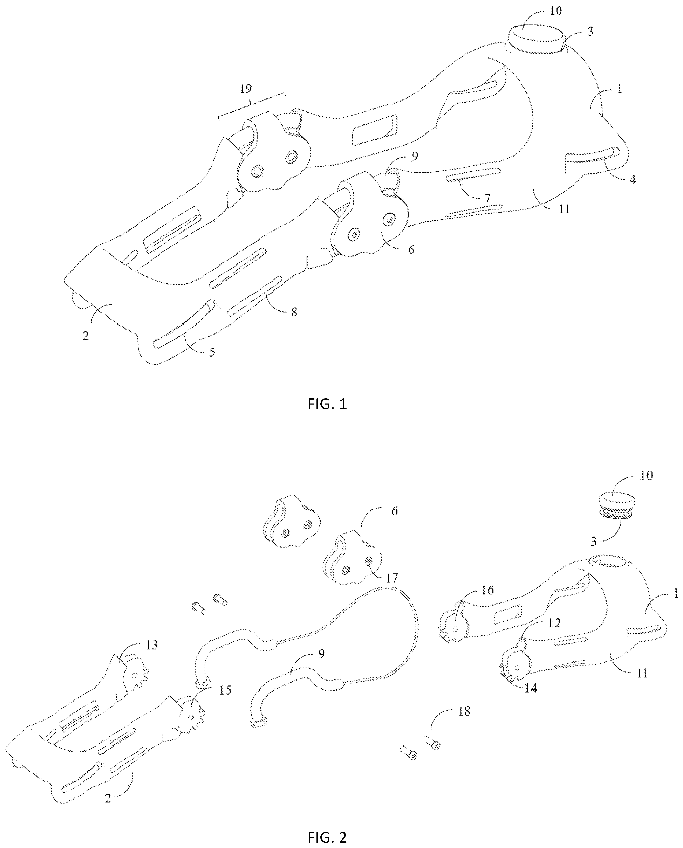

**Table 1,** *infra,* lists the components illustrated in FIG. **1** for the knee brace frame. The knee brace comprises a knee brace frame **11,** or vertical support, a geared pivoting hinge assembly **19,** and an adjustable tensioning mechanism **3.** As illustrated in FIG. **1****,** the vertical support comprises: an upper (proximal) frame **1,** and a lower frame **2.** In this particular embodiment, both portions **1** and **2** fit to the front side, or anterior surface, of a user's leg just above and below their knee. In an embodiment, the knee brace frame **11** is sized small, medium, or large, depending upon the outer circumference of the user's thigh; or, the knee brace is custom designed and fabricated to fit a specific patient's knee, which can be performed by an electric digital scan. Normally, the diameter and circumference of the upper frame **1** is larger than that of the lower frame **2.**

*Straps:* In aspects, the brace comprises straps. In this embodiment, the vertical support further comprises on the back, posterior side, of the knee brace **11,** at least one horizontal strap above and below the knee to secure the brace to the user's leg. In one embodiment, two external loops exist on the posterior side of the upper frame and two external loops exist on the posterior side of the lower frame, wherein each frame has a loop on both the medial and lateral sides. At least one strap extends horizontally on the posterior side of the brace **11** between the brace upper portion **1's** medial and lateral side and within the upper frame external loops **4,** and at least one strap extends horizontally between the brace lower cuff **2**'s medial and lateral side and within the lower frame external loops **5.**

**Table 1- Knee Brace 11**

| **FIG. Item #** | **Component Name** |
|---|---|
| 1 | Upper frame |
| 2 | Lower frame |
| 3 | Adjustable tensioning mechanism |
| 4 | Upper frame external posterior loops |
| 5 | Lower frame external posterior loops |
| 6 | Center cap |
| 7 | Upper frame internal strap loops |
| 8 | Lower frame internal strap loops |
| 9 | Tensioning element |
| 10 | Adjustable tensioning knob |
| 19 | Hinge assembly |

### Hinge Assembly

Table 2 lists the components illustrated in FIG. **2** for the hinge assembly. In aspects, the present disclosure comprises at least five different pivoting hinge assemblies, in aspects comprising at least one tensioning element **9,** and two geared teeth, comprising a proximal gear **14** and a distal gear **15.** Each type of hinge assembly can be used to generate tension in a one-sided brace (hinge medial or lateral side) or a full knee brace (hinge medial and lateral sides). In embodiments, the hinge assembly proximal end is connected to the brace upper frame **1,** and the hinge assembly distal end to the lower portion **2,** or in a similar manner to a variety of knee braces known in the prior art for unloading weight from the knee joint.

The two opposing gears **(14, 15)** of the hinge assembly **19,** are connected via a center cap **6;** the frame has a proximal opening **12** and a distal opening **13** that houses the tensioning element **9** and allows it to stretch across the intermeshing gears, resisting flexion. The teethed gears have a central hole **16,** and the center caps have holes **17** in line with the gear central holes **16,** which are functionally attached to a center core bracket **6** to allow for rotation around the gears while generating tension (or a breaking force, or a counter-restorative force), thus allowing the wearer of the brace to more easily flex and extend. The gears and the brackets may be functionally attached using screws, bolts, or another method known in the art **18.** The center core brackets are positioned medial and lateral to the subunits, and are able to function to: pin the subunits together while enabling the gears to rotate in unison; protect the gears and tensioning element; and limit a maximum degree of flexion of the hinge assembly. In another embodiment, the element may be stretched under the gears, to assist with flexion; this may be used in a brace that is designed to help rehabilitate the knee after an injury.

The teethed gears further provide a mechanism to limit the maximum extension of the tensioning elements and hinge assembly to prevent hyperextension of the knee using extension and flexion stops and, in aspects, radially oriented slots. The slots allow for insertion of extension flexion stops, which are pre-made inserts that restrict the range of motion of the joint. The extension and flexion stops will not permit the gears or hinge to rotate further once contact is made with the stops. The allowable surface angle between the gears' point of contact is a design variable that can be modified to satisfy user requirements. Additionally, the extension and flexion stops may be designed in a way not present in the drawings; for example, the extension and flexion stops can be designed to fit between the gears on either the posterior or anterior side of the hinge assembly in order to limit the range of motion of the join.

**Table 2-Hinge Assembly 19**

| **FIG. Item** # | **Component Name** |
|---|---|
| 12 | Upper frame proximal thread hole |
| 13 | Lower frame distal thread hole |
| 14 | Proximal teethed gear |
| 15 | Distal teethed gear |
| 16 | Teethed gear central hole |
| 17 | Center cap hole |
| 18 | Fastener |

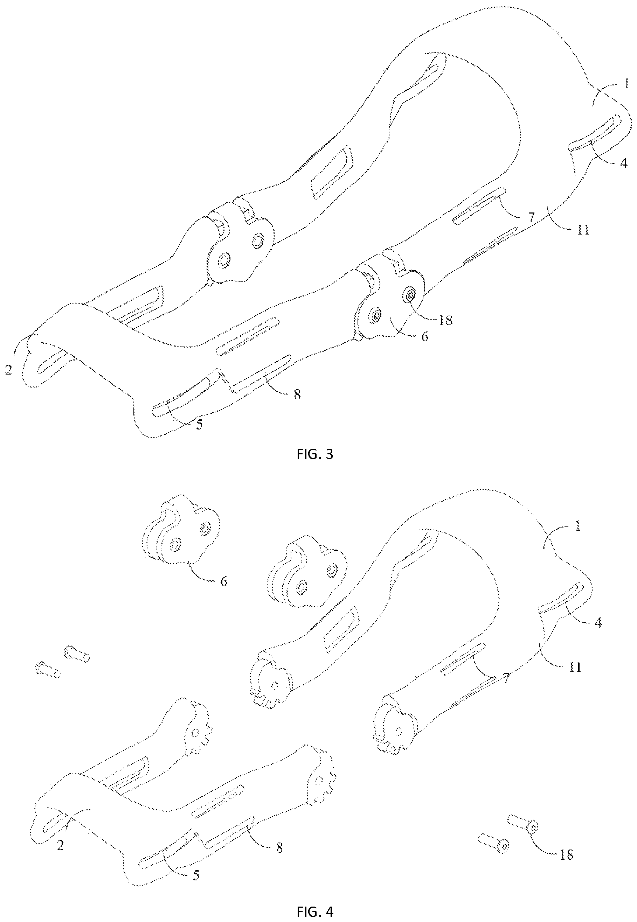

The hinge assembly may incorporate at least one tensioning element **9** that is attached on each side of the upper and lower hinge assembly. The tensioning element stores energy when it is drawn across the hinge upon knee flexion by the wearer of the knee brace. The tensioning elements are ported through holes in the hinges **(12, 13)** or support members and are fixed in place in the brace on either the proximal or distal end, or both. Alternate designs are based on the needs of the user and include one or multiple tensioning elements within the hinge assembly on either or both the medial or lateral side of the knee brace, or above or below the hinge. These alternate designs also include bands of varying sizes that generate different amounts of resistance. Using more than one band can be tailored to engage and increase in tension as the degree of flexion is increased. An embodiment of the invention that does not include an adjustable tensioning system is illustrated in FIG. **3-4****.**

In aspects, the device described may be a 3-D printed brace that includes either or both a medial or lateral unloader feature using a three point system of pressure or force where pressure or force is applied on the lateral side, for example, and pressure or force is applied on the lateral side, above the knee and below the knee, to generate a distraction force between the tibia and the femur in the medial compartment, as an example. this device will include patient specific information that will enable the amount of unloading force or pressure to be appropriately assessed and exerted on the patient based on their need. The degree of medial or lateral unloading, in aspects, can be adjustable by the user via a knob, slide, button, tab, digital screen, processor, controller, motor, microdrive, switch, pulley, block and tackle system, or lever, that the wearer can use to adjust an amount of medial or lateral force applied to the joint. For example, a screw mechanism in the hinge may be adjusted to push the medial condyle pad into the knee, providing a corrective force.

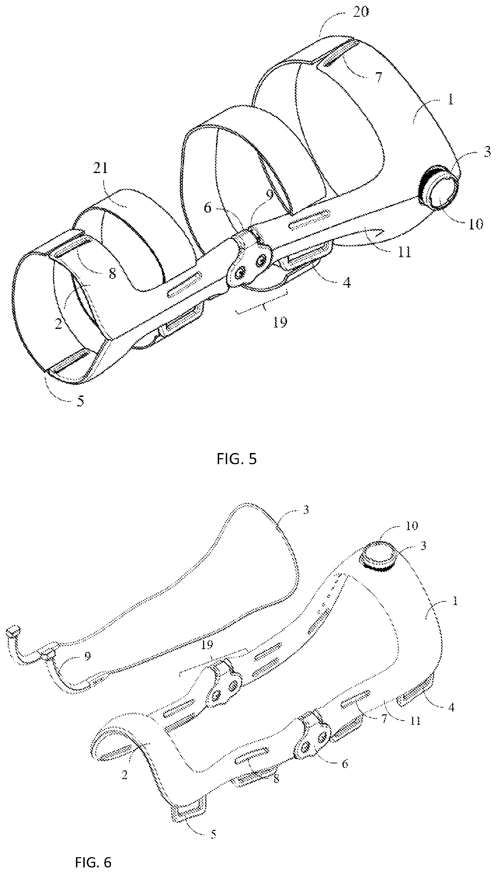

As seen in FIG. **5****,** a single upright knee brace may include a tensioning system 3. A single upright knee brace may provide valgus or varus unloading support by utilizing material flexibility, rigidity, and strength characteristics to apply forces in the desired location(s). For example, a brace designed to treat varus to relieve medial compartment osteoarthritis may apply a lateral or medial force in the center of the brace at the hinge assembly **19.** The curvature of the frames **(1, 2)** away from the leg will apply a medial or lateral force above and below the user's knee, resulting in a corrective or distractive force that will reduce pressure in the medial or lateral compartment.

The single upright brace frame is comprised of a proximal frame **1** and distal frame **2** connected with a center cap **6.** In aspects, a combination of rigid or semi-rigid proximal and distal supports **(20, 21)** are required. In aspects, the brace can be worn on the medial or lateral side of the leg. In aspects, the brace can treat varus or valgus from either the inside or outside of the leg. For the unloading variant, the tensioning element **9** may be drawn over the hinge and tensioned using one or more elastic bands and a tension-locking mechanism **10** such as a BOA dial or other tension-adjusting mechanism that may be placed on the upper **1** or lower portion **2** of the frame. The brace may be contoured around the leg and patella to prevent or minimize rotation around the leg and migration down the leg with rigid or semirigid supports **(20, 21).** An alternative version of this brace has a fixed tensioning element. A strapping system may be incorporated into the brace frame **(20, 21).**

As seen in FIG. 6, an alternative system involves a mechanism where one or more tensioning elements **9** are integrated within the upper portion **1** or lower portion **2** of the brace frame rather than drawn over the hinge. These bands may occur as one or more and may be found in either or both the upper and lower members. The brace frame may involve a tensioning system **3** that controls the amount of torque around the hinge **19.**

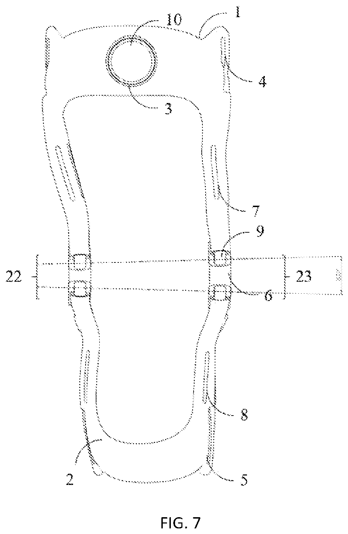

An embodiment of the invention that includes hinge assemblies **(22, 23)** with gears of variable radii hinge as a means of distraction is seen in FIG. **7-8****.** The proximal and distal gear radii **(24, 25)** on one side of the brace and can differ from the gear radius on the other side **(26, 27).** This means of distraction is also applicable for a single upright knee brace.

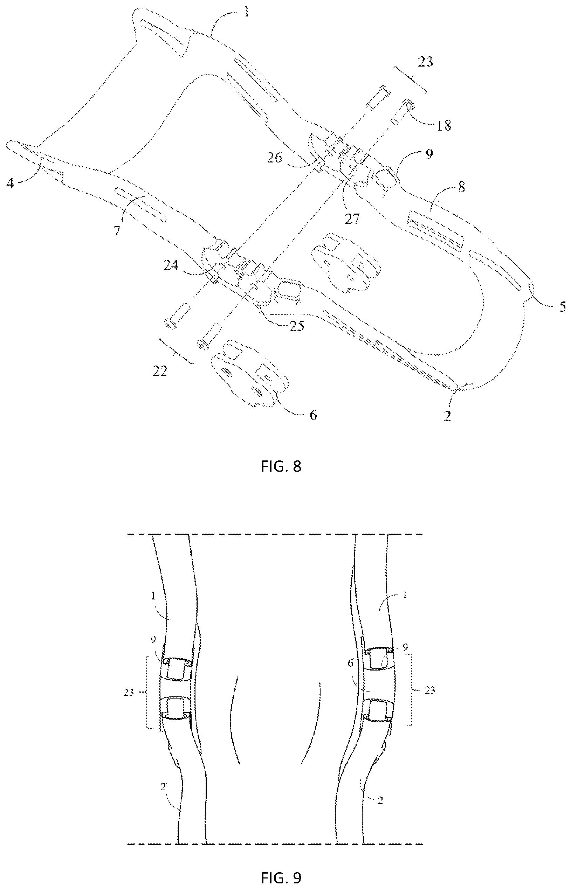

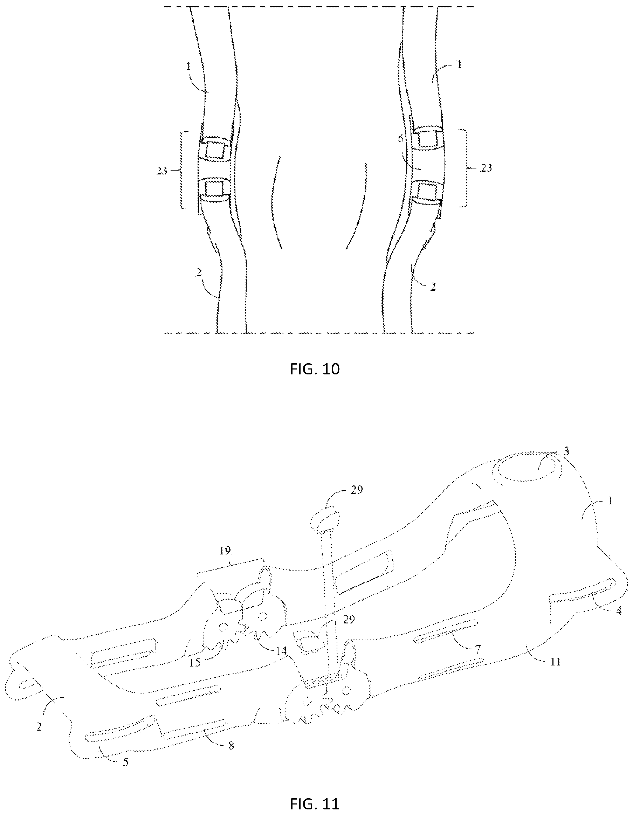

As seen in FIG. **9****,** an embodiment of the invention includes a conforming hinge assembly **23** that may be tailored to achieve a desirable loading profile. The conforming hinge assembly may be comprised of proximal and distal gears, cam inserts, and hinge caps. The assembly may be curved to fully or partially conform to the user's joint or limb as opposed to a flat shape which will generate an increasing amount of tension per degree of flexion and the tensioning element **9** will travel further over the cam or gear mechanism. This embodiment may have an adjustable tensioning element as seen in FIG. **9** or no tensioning element as seen in FIG. **10****.**

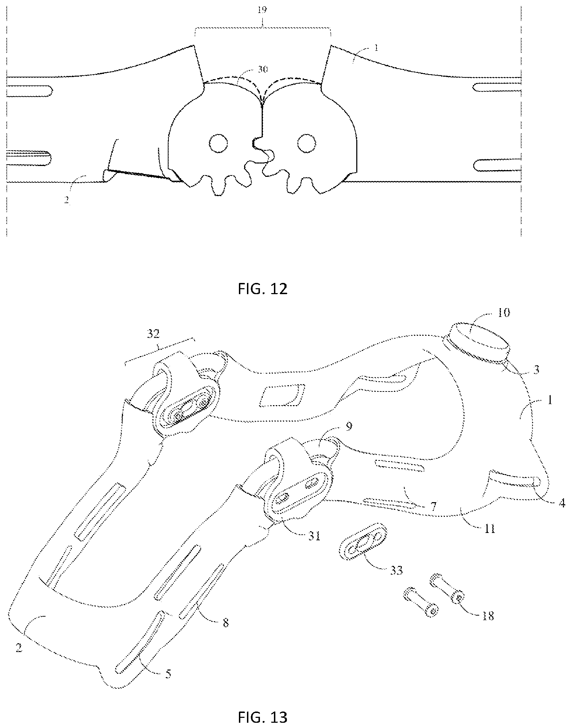

As seen in FIG. **11****,** the subunits may further comprise cam units **29,** e.g. located within the subunits as carved or molded into the internal housing of the subunits and residing slightly above-anteriorly to the gears so that the tensioning elements are drawn over the cams and the gears. The cam units increase the tension in the tensioning elements with increasing degrees of flexion of the user's knee. The cam geometry is variable and designed to generate a force that corresponds to unloading requirements. Alternatively, the cam profiles **30** may be manufactured and operatively embedded into the proximal and distal gears using 3D printing techniques as seen in FIG. **12****.**

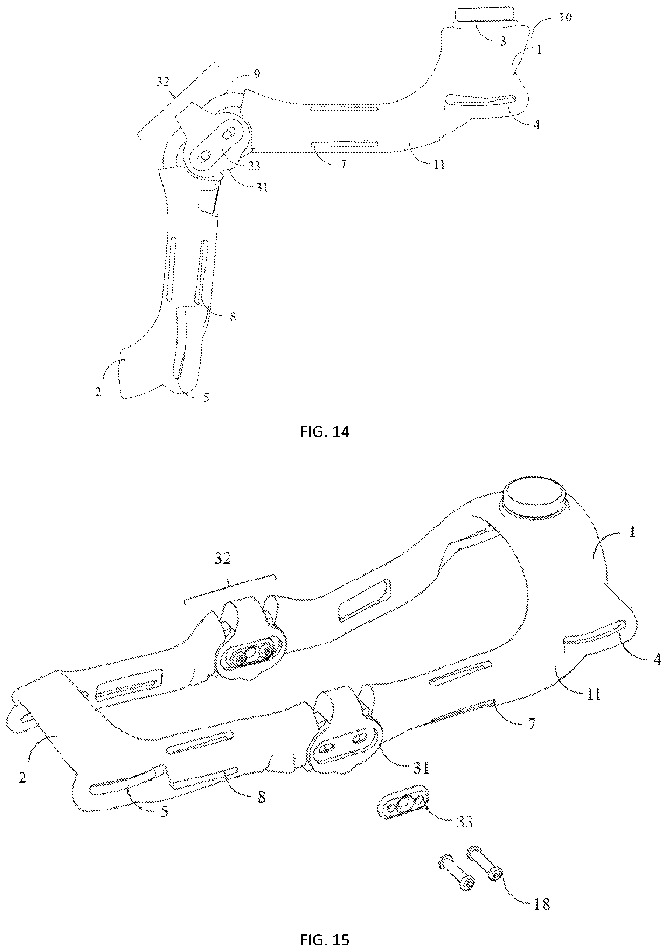

An embodiment of this invention to create joint distraction involves a slot mechanism with a distraction hinge assembly **32** comprised of a distraction hinge cap **31** and distraction hinge center core bracket **33** as seen in FIG. **13** when the knee brace is in extension and FIG. **14** when the knee brace is in flexion. One or more slots in the center core bracket **33** circumscribe a pivot point of the hinge **32** which allows for limited direction of travel based on gear radius. The slots in the center core bracket **33** determine the direction of the distraction A center cap **31** encases the hinge and may contain slots to support the hinge mechanism. One or more fasteners **18** in the form of bolts, screws, or similar attachment mechanisms connects the center cap **31** to the upper **1** and lower portion **2** of the brace frame, and the center core bracket **33** or cap **31** comprises slots allowing the upper and lower portion to translate. This embodiment may or may not include an adjustable tensioning system. FIG. **15** illustrates one aspect of the distracting hinge mechanism with fixed tension.

Embodiments of the invention that include one or more sensors on the device, and in aspects a processor on or off the device, also include a computer readable medium comprising one or more computer files comprising a set of computer-executable instructions for performing one or more of the calculations, steps, processes and operations described and/or depicted herein. In exemplary embodiments, the files may be stored contiguously or non-contiguously on the computer-readable medium. Embodiments may include a computer program product comprising the computer files, either in the form of the computer-readable medium comprising the computer files and, optionally, made available to a consumer through packaging, or alternatively made available to a consumer through electronic distribution. As used in the context of this specification, a "computer-readable medium" is a non-transitory computer-readable medium and includes any kind of computer memory such as floppy disks, conventional hard disks, CD-ROM, Flash ROM, non-volatile ROM, electrically erasable programmable read-only memory (EEPROM), and RAM. In exemplary embodiments, the computer readable medium has a set of instructions stored thereon which, when executed by a processor, cause the processor to perform tasks, based on data stored in the electronic database or memory described herein. The processor may implement this process through any of the procedures discussed in this disclosure or through any equivalent procedure.

In other embodiments of the invention, files comprising the set of computer-executable instructions may be stored in computer-readable memory on a single computer or distributed across multiple computers. A skilled artisan will further appreciate, in light of this disclosure, how the invention can be implemented, in addition to software, using hardware or firmware. As such, as used herein, the operations of the invention can be implemented in a system comprising a combination of software, hardware, or firmware.

Embodiments of this disclosure include one or more computers or devices loaded with a set of the computer-executable instructions described herein. The computers or devices may be a general purpose computer, a special-purpose computer, or other programmable data processing apparatus to produce a particular machine, such that the one or more computers or devices are instructed and configured to carry out the calculations, processes, steps, operations, algorithms, statistical methods, formulas, or computational routines of this disclosure. The computer or device performing the specified calculations, processes, steps, operations, algorithms, statistical methods, formulas, or computational routines of this disclosure may comprise at least one processing element such as a central processing unit (i.e., processor) and a form of computer-readable memory which may include random-access memory (RAM) or read-only memory (ROM). The computer-executable instructions can be embedded in computer hardware or stored in the computer-readable memory such that the computer or device may be directed to perform one or more of the calculations, steps, processes and operations depicted and/or described herein.

Additional embodiments of this disclosure comprise a computer system for carrying out the computer-implemented method of this disclosure. The computer system may comprise a processor for executing the computer-executable instructions, one or more electronic databases containing the data or information described herein, an input/output interface or user interface, and a set of instructions (e.g., software) for carrying out the method. The computer system can include a stand-alone computer, such as a desktop computer, a portable computer, such as a tablet, laptop, PDA, or smartphone, or a set of computers connected through a network including a client-server configuration and one or more database servers. The network may use any suitable network protocol, including IP, UDP, or ICMP, and may be any suitable wired or wireless network including any local area network, wide area network, Internet network, telecommunications network, Wi-Fi enabled network, or Bluetooth enabled network. In one embodiment, the computer system comprises a central computer connected to the internet that has the computer-executable instructions stored in memory that is operably connected to an internal electronic database. The central computer may perform the computer-implemented method based on input and commands received from remote computers through the internet. The central computer may effectively serve as a server and the remote computers may serve as client computers such that the server-client relationship is established, and the client computers issue queries or receive output from the server over a network.

The input/output interfaces may include a graphical user interface (GUI), which may be used in conjunction with the computer-executable code and electronic databases. The graphical user interface may allow a user to perform these tasks through the use of text fields, check boxes, pull-downs, command buttons, and the like. A skilled artisan will appreciate how such graphical features may be implemented for performing the tasks of this disclosure. The user interface may optionally be accessible through a computer connected to the internet. In one embodiment, the user interface is accessible by typing in an internet address through an industry standard web browser and logging into a web page. The user interface may then be operated through a remote computer (client computer) accessing the web page and transmitting queries or receiving output from a server through a network connection. Additionally, in aspects, the brace will allow the user to interact with it using other interfaces, such as, but not limited to, foot pedals, physical buttons, haptic feedback, or projected interface elements, and may include multiple interface options in combination with one another, to allow maximum flexibility in the ways the user can interact with the brace.

Although the above-recited examples are not to be construed as limiting the scope of the various embodiments of the present disclosure, the examples indicate that the knee brace and hinge assemblies can be constructed for use in an elbow brace. It is apparent that the skilled artisan can modify the dimensions of the brace and hinge assemblies to treat pain and inflammation associated with a variety of elbow disorders. The same is true of the ankle and other joints.

It is also readily apparent that the range of adjustability of the braces within the scope of the present invention *inter alia* by selecting materials of different elasticity for construction of the arm members, by selecting different longitudinal or cross-sectional dimensions for the arm members, or by selecting pads of different fixed thicknesses or different ranges of adjustable thicknesses.

It is further evident that although the knee brace and hinge assemblies of the present invention have only been described above in terms of a few embodiments adapted to treat osteoarthritis, it is apparent to the skilled artisan that these embodiments are readily adaptable to treatment of pain associated with a variety of knee disorders. For example, additional embodiments envisioned with the scope of the present disclosure comprise hinge assemblies with the user tension adjustment handle, knob, etc. on the user's tibia versus the exemplified embodiment on the user's femur.

The invention provides for a manufacturing method starting, in aspects, from a biological body study until fabrication. An example workflow is followed; however, the stages may be reordered, skipped, or modified depending on the application.

While knee braces and orthotics are used by example to describe embodiments of the invention, one skilled in the art would understand that the following descriptions could be applied to any orthotic, prosthetic, wearable device, or user interface (e.g., a handle, vehicle seat, controller, etc). Further, the current invention as described herein could not only be applied to knee braces or orthotics, but could also be used for neck, shoulder, hip, wrist, handle, ankle, and foot applications, by way of example only. Furthermore, the current invention could be applied to wearable technologies and accessories including custom performance wear, watches, helmets, military gear, and footwear by way of example only.

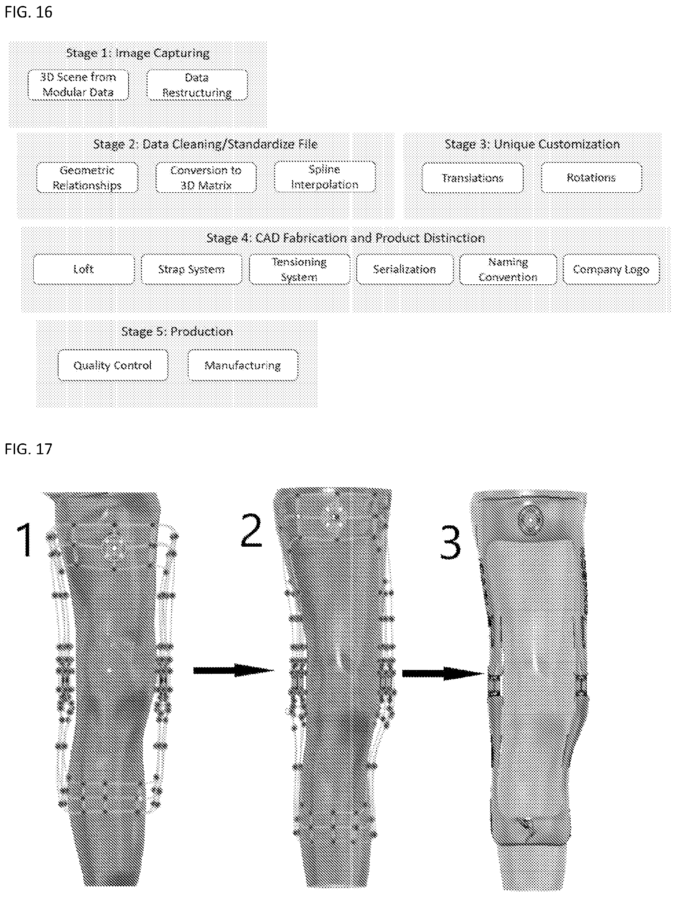

An exemplary embodiment of the process outlined below is represented in Figure 16.

### Stage 1:

For a desired part or parts of the body for which the device, wearable or user interface is being designed, data is collected, which is then processed to render a 3D digital object. In embodiments, this object may be generated using a 3D scan on a device such as a phone, depth camera, tablet computer, or other computer device. The 3D digital object represents a surface to which a device may be relatively fit or contoured. The 3D digital object may be generated indirectly, for example making a cast of the body part and then scanning the cast. The 3D digital object may be generated based on data representing at least 4 points in space (any number of data points that could represent a 3D object or space) or measurements. For example, circumference measurements of the thigh and calf taken manually by a physician may be input into a system, which will render a 3D leg based on a formula of prior measurement data in combination with or independently from 3D scan data or 2D pictures.

In aspects, internal body components (e.g., bones, tendons, cartilage) may be digitally rendered or captured using the 3D scan alone or in addition to other technologies, such as ultrasound, magnetic resonance imaging, x-ray, or scan. In aspects, internal body components may be digitally rendered as a function of the 3D scan data, measurements, or in reference to the 3D digital object.

3D digital objects may be captured through a range of motion to capture the envelope of motion of a joint (e.g., the knee from full flexion through full extension or an ankle in multiple axes of rotation).

Additional data may be collected and input into the system manually or automatically. Such data may include quantitative data from a prescribing doctor including Q angle of the knee, required varus or valgus correction, required range of motion limitation or augmentation, required support or assistance (e.g. unloading that may be provided by a device that generates force in one or multiple directions). Such data may include patient biometric data or user data such as BMI, age, gender, height, weight, current activity level, desired activity level, and mechanical impedance or elasticity of a part of the body. Such data may include patient qualitative data such as degree of patient pain, location of pain, pain during movement or different activities, pain throughout a range of motion, and desired device use, user activity, or lifestyle.

Further data may be indirectly generated based on initial data inputs. For example, the degree of elasticity/mechanical impedance of the thigh, calf or other body part may be extrapolated from BMI and/or age based on previously defined functions. This would allow for automated design of a device that has dimensions or mechanical properties to provide more compression or a tighter fit in the thigh region, allowing for a better fit, less sliding, and improved performance.

Radiographic data may be included to instruct on device shape for corrective purposes to restore healthy joint geometry or unload a region of the joint. In embodiments, data collection may include individually or combinations of Magnetic Resonance Imaging (MRI), Computed Tomography (CT) scan, Position Emission Tomography (PET) scan, X-ray, fluoroscopy or ultrasound data. Such data may reveal cartilage degeneration or osteoarthritis in a joint, inflammation, deformity or damage of bone, cartilage, muscle, tendon, ligament, nerves, skin/epithelium or other tissue, that would automatically or semi-automatically inform device design to correct joint geometry, prevent undesired or unnatural motion, enhance desired motion, or bias the movement of a joint to reduce user pain, for example during gait or a specific activity.

Objects may be represented as a mesh or other digital file format, a matrix of points, a series of splines or a stack of 2D geometries among other representations to form a 3D digital object.

The data is either transferred to a larger database (e.g., external hard drive, server, or the cloud) or used/stored on the capturing device. During the transferring of files, the data may or may not be converted into a new file format. The file may be converted to or from the following formats, however, this list is non-exhaustive: ply, stl, obj, and usdz file extensions. For example, a 3D scan collected on a smartphone app may be stored in a cloud database as a usdz file, which may be further converted to an obj file that becomes the reference object for design in Stage 2.

### Stage 2:

The following stages may be done in any particular order. An example is illustrated below. The data file from stage 1 may be cleaned to remove irregularities, soften extrema, connect surfaces, or modify locations. Cleansing may take the form of removing, adding or joining points, lines, planes, or surfaces. Data compression may also occur to improve overall efficiency, reduce operation time, and minimize required computing power.

In embodiments, within a computer-aided design ("CAD") file, one or more reference objects have specific geometric constraints. An example of a file would include a multitude of cross sections serving as a fluid wireframe of the device. Additional lines, acting as rails, would run through a specific point within each cross section to define the bounds of the device. These geometries can serve as a standard baseline for the shape of the device, allowing for automatic adjustment of this file given the inputs from stage 1. Additionally, 3D objects may also exist within the standardized file if those objects will be added to the final customized device. An example would be using a barrel bolt to connect separate parts of the device. Another example would be using the same sub-component in any version of said device such as a gear that is present regardless of the input data. These reference objects can be preset, generated, or modified based on the input data from stage 1. An algorithm may set the geometric constraints of each object based on the input provided and direct or indirect reference to other objects, data points, surfaces, or splines within the file. For example, a cross-section of the device may be grounded (e.g., unable to move or rotate in any coordinate direction) for a specific design constraint regardless of the position of the mesh. The algorithm's specific transformations, including rotation, translation, mirroring and isotropic or anisotropic scaling for fitment, will be overruled by the geometric constraint and that specific object will remain unmoved. In aspects, the geometric constraints limit the translation by modifying vector components in the *eᵢ* , *e_{f}, eₖ* directions---where "e" represents an orthogonal axis in some coordinate system. Additionally, the geometric constraints may limit rotation around any axis by modifying components within a three-dimensional matrix transformation or other data format which stores and/or modifies the orientation of an object.

### Stage 3:

Once a reference object (143) possesses geometric constraints, transformations including translations (145-147), rotations (148-510), mirroring, and/or isotropic or anisotropic scaling are performed to move a reference object into a new location. These movements may be determined by the data illustrated in Stage 1. Transformations may also be applied to reference objects as a whole or in part to optimize fitment. After one or more reference objects are in the correct location, the algorithm described in Stage 2 may be reapplied with the new location of the reference objects as an input. As such, the objects will be translated, rotated, transformed or scaled relative to the geometric constraints as defined by the data of Stage 1. For example, the geometry and fit of a top brace frame (104-108) may be defined by multiple objects that are further defined by the geometric constraints. An object defining the position of a rotary element (117, 123) may be positioned relative to the top frame and combined with or separated from one or multiple parts of the device based on these constraints and the characteristics of the base device.

In embodiments, the base device may be divided into two or more component parts prior to, during, or following modifications. The components may then undergo further modifications and transformations. For example, a knee brace base device may be divided into a top frame and a bottom frame, with an articulating hinge interface. As another example, a base device for an ankle orthosis may be transformed as one complete unit to fit the user's ankle, heel and foot. The file may then be divided into a custom fit ankle cuff and a custom fit foot orthotic, which may be attached by other components during assembly.

In other embodiments, qualitative and quantitative data from physicians or patient surveys input in Stage 1 may lead to modifications of the device. For example, an individual indicative pain on the medial side of the knee with a recommended Q angle correction of 3° would adjust the brace frame for a correction to unload the medial compartment of the knee using the positioning of objects such as specific cross sections of the frame or gears.

In other embodiments, radiographic information may lead to automated adjustment of the device. For example, the identification of arthritis in the medial compartment of the knee may trigger a similar brace frame adjustment as described in the previous example.

In other embodiments, patient data, including biometric data, may lead to automated adjustment of the device. For example, for an individual with an above average BMI, the top brace frame may be adjusted for a tighter fit and greater compression of the adipose tissue in the thigh.

In other embodiments, scan data showing envelope of motion for a joint may adjust design to optimize the axis of rotation of a device throughout a range of motion. The adjustment may limit or augment movement of the joint or body point in a given direction based on a gait pattern in combination with or independently from data from the physician or radiographic data. For example, the length of a slot in a hinge through which a bolt travels during brace flexion and extension may be limited to reduce the travel of the bolt, thereby limiting the user to a range of motion of 20° to 125° of flexion.

### Stage 4:

Whether directly modifying the CAD software or through an application programming interface (API), a series of one or more three-dimensional modeling techniques can be applied to a base device to create the manufacturable device. One or more geometries or features may be generated and added to the fitted model at this stage. Additionally, both additive and subtractive techniques may be applied to add in individual components or subsystems to the device. These subsystems may include a strap system (130-133 and 134-411), tensioning system (26), and resistance system or any system necessary for the application of the device. In embodiments, the types of bodies generated automatically or semiautomatically may be based on the type of device or corresponding base device of the program. For example, a product identified as knee brace type 2 model 3 could generate 4 D-rings (130-133), 8 strap slots (134-141), a boa insert on the upper frame (126) and a mechanical gear interface (128-129). The shape, dimensions, positioning, orientation and design of the component may be defined based on the base device. In embodiments, the characteristics of the component will be defined directly or indirectly as a function of the data collected in Stage 1, including 3D scan data, patient data, radiographic data, and intended use data. The component may be further characterized directly or indirectly as a function of the geometric constraints and objects defined in Stage 2. In other embodiments, the characteristics of the components will be defined relative to or as a function of the dimensions, positioning, or curvature of all or part of the modified base device generated in Stage 3. The components may be modified in groups (each component within the group experiencing the same modification), subgroups, or as individual components. One type of modification, for example scaling and dimensions, may be applied uniformly and simultaneously to a group before individual components within that group go through a separate type of modification (e.g. rotation), which may provide improvements in the efficiency or accuracy of the program. In embodiments, the components may be generated or modified in stages and characteristics defined as a function of other components previously generated.

The modified base file and components may be further modified to alter the shape, surface curvature, texture, printing pattern, or infill pattern. The modifications may be applied in part or in whole. The modifications may impart beneficial mechanical properties at regions of the device. For example, a cross sectional geometry may be applied in a region of the device that will experience a higher degree of compression in one or multiple directions to provide greater durability under load. This may also optimize the device's overall strength to weight ratio. The infill density or infill pattern may be automatically adjusted in regions of the device for similar purposes. The infill pattern, density, or other modifications may be performed only on select components or on the combined base device and components (the whole device) in a predefined gradient. It may be defined or applied based on geometric properties or constraints, such as thickness of the part at a given cross section, relative position from a point, spline, object or other geometric feature. In embodiments, it may be defined/applied based on analysis of the strength or flexibility of the object, which may be determined dynamically or at a specific stage of the process with mechanical analysis such as finite element analysis.

Parts of the base device, components, or the interface between components and the base device or between components themselves may be further modified with techniques including but are not limited to extrusion, combining, lofting, sculpting, and rotations. Techniques (e.g., fillets, chamfers, and holes) may be applied to the digital representation of the manufacturable device to convert the base device into a usable product. Modifications from such techniques may further improve the fit of a device to a body part or improve the strength of the device overall. For example, fillets may be added at the interface of components, such as D-rings or strap slots, with the base device to eliminate stress concentrations. Filets may be applied throughout the device in one or multiple automated stages that eliminate sharp angles or interfaces to eliminate stress concentrations, improve strength at critical failure points, and improve the device's strength overall. Filets or surface smoothing may also be applied to areas of the device that will contact the skin, which may be determined by the proximity of parts of the device to the 3D digital object (e.g., leg scan). This can eliminate sharp edges to maximize comfort for the user.

After the device's main form is created, product distinction techniques may be applied. This may include serial numbers, custom names, custom engravings, company logo engravings, and company name engravings. Such distinction features may be automatically generated based on the initial data input, the geometric constraints, or the to identify type, properties, or sequence of the device in a manufacturing batch.

### Stage 5:

Lastly, a quality control element of the automation may be applied. This element may include checking bounds of mass, the bounds of size, the curvature of the body, continuity of bodies within the device, finite element analysis for strength, and bounds of clearance values to ensure the device meets all specifications needed for manufacturing. After the quality control, the CAD file may be converted to a new file type for manufacturing. File types may include but are not limited to: STL, OBJ, VRML, and X3G. The file may or may not be sent to a new database or used on the current device. The file can then be converted into a physical model of the device using either additive or subtractive manufacturing techniques.

With the currently described autonomous vertical integration herein, one of the most important improvements is time to produce the device. According to the present invention, the consumer may receive the necessary device on a significantly shortened time frame. Due to the current invention, devices can now be produced and purchased at a lower cost. This allows for a larger market to interact with the object--improving the quality of life of a larger populus.

The device can be created better with the described algorithm than any individual or group of individuals. Being able to mathematically decide the form based on tangibles creates a superior device and eliminates the possibility of any human error during device creation. Superiority comes in the form of improved fitment, increased comfort, greater ultimate strength, and a larger resistance to shearing forces.

Lastly, the automation allows for a consistent device. Despite being custom, a wearer will be able to accurately predict the interaction the device has with his or her body. Another benefit of the consistency is the removal of human error. The device will be seamless and ensure the wearer does not wait longer to address the needs that would be fulfilled by the product.

In application, by way of an example, a patient with multicompartment knee OA, including patellofemoral and medial compartment OA, would visit a doctor after experiencing knee pain during activity. The patient may also consult a doctor remotely using telehealth. The doctor would identify indications of knee OA, including reduced stability, user pain during specific activities such as climbing stairs, and potential joint deformity. At this stage, a custom brace may be prescribed. The doctor, another health professional, or the user may scan the joint using software on a phone or tablet. The scan data would then be uploaded to a cloud-based server. Data may be cleaned, processed, or converted into a different file type on the device used to take the scan or after being transferred to the server. The prescriber may also provide supporting radiographic data, such as an MRI, through the health network's user interface, which may then be sent to the same or a different cloud-based server. Data from the same server or different servers within the network may be paired by a unique identifier, such as a patient or job ID number. Quality analysis of the data may be performed either on the device or user interface where the data is collected or in the server on which the data is stored. The user or doctor may receive error messages indicating that data needs to be recaptured if it does not pass quality standards.

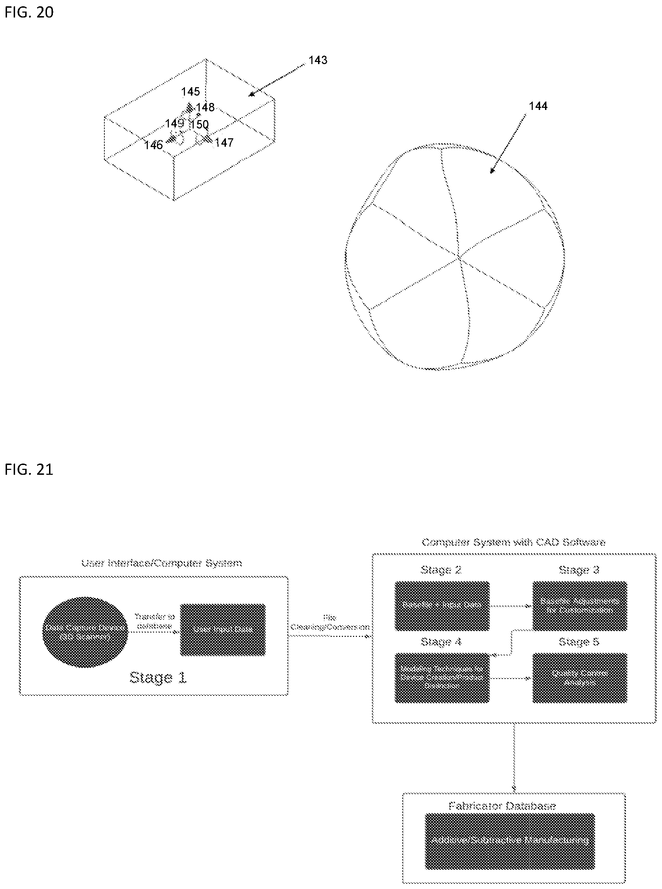

Processed data will then be transferred to a computer system on which the software comprising the code for operations from Stages 2 through 5 are performed (as described above). An operator may oversee the process as performed by the software. The software may present them with reports, in real time or at different stages of the process to assess the accuracy and progress of the device modeling. The operator may approve performance of different functions between or within different stages of the process for the software to proceed in modeling. Alternatively, they may opt for corrective action, manually or through activation of a different code/software function, if the quality of device modeling is deemed insufficient based on reports or observation. Once the device has been fully formed, quality control analysis in stage 5 may be implemented on the same computer system or a separate system to achieve a final, approved device for fabrication. The modeling system may be integrated with the fabrication system, which controls 3D printing output, through an API to automatically queue and print the digitally represented device. The final device may be fabricated as one continuous part from one or more materials. Alternatively, additional components may be added during an assembly process to yield a final device. The entire process, from data collection to delivery of a final, custom fit device to fulfill the user need may be completed, in aspects, within 12 hours based on the current, automated system as described. A fully automated system that performs the above process is exemplified in Figure 21.

Embodiments of the invention also include a computer readable medium comprising one or more computer files comprising a set of computer-executable instructions for performing one or more of the calculations, stages, processes and operations described and/or depicted herein. In exemplary embodiments, the files may be stored contiguously or non-contiguously on the computer-readable medium. Embodiments may include a computer program product comprising the computer files, either in the form of the computer-readable medium comprising the computer files and, optionally, made available to a consumer through packaging, or alternatively made available to a consumer through electronic distribution. As used in the context of this specification, a "computer-readable medium" is a non-transitory computer-readable medium and includes any kind of computer memory such as floppy disks, conventional hard disks, CD-ROM, Flash ROM, non-volatile ROM, electrically erasable programmable read-only memory (EEPROM), and RAM. In exemplary embodiments, the computer readable medium has a set of instructions stored thereon which, when executed by a processor, cause the processor to perform tasks, based on data stored in the electronic database or memory described herein. The processor may implement this process through any of the procedures discussed in this disclosure or through any equivalent procedure.

In other embodiments of the invention, files comprising the set of computer-executable instructions may be stored in computer-readable memory on a single computer or distributed across multiple computers. A skilled artisan will further appreciate, in light of this disclosure, how the invention can be implemented, in addition to software, using hardware or firmware. As such, as used herein, the operations of the invention can be implemented in a system comprising a combination of software, hardware, or firmware.

Embodiments of this disclosure include one or more computers or devices loaded with a set of the computer-executable instructions described herein (Figure 21). The computers or devices may be a general purpose computer, a special-purpose computer, or other programmable data processing apparatus to produce a particular machine, such that the one or more computers or devices are instructed and configured to carry out the calculations, processes, stages, operations, algorithms, statistical methods, formulas, or computational routines of this disclosure. The computer or device performing the specified calculations, processes, stages, operations, algorithms, statistical methods, formulas, or computational routines of this disclosure may comprise at least one processing element such as a central processing unit (i.e., processor) and a form of computer-readable memory which may include random-access memory (RAM) or read-only memory (ROM). The computer-executable instructions can be embedded in computer hardware or stored in the computer-readable memory such that the computer or device may be directed to perform one or more of the calculations, stages, processes and operations depicted and/or described herein.

Additional embodiments of this disclosure comprise a computer system for carrying out the computer-implemented method of this disclosure. The computer system may comprise a processor for executing the computer-executable instructions, one or more electronic databases containing the data or information described herein, an input/output interface or user interface, and a set of instructions (e.g., software) for carrying out the method. The computer system can include a stand-alone computer, such as a desktop computer, a portable computer, such as a tablet, laptop, PDA, or smartphone, or a set of computers connected through a network including a client-server configuration and one or more database servers. The network may use any suitable network protocol, including IP, UDP, or ICMP, and may be any suitable wired or wireless network including any local area network, wide area network, Internet network, telecommunications network, Wi-Fi enabled network, or Bluetooth enabled network. In one embodiment, the computer system comprises a central computer connected to the internet that has the computer-executable instructions stored in memory that is operably connected to an internal electronic database. The central computer may perform the computer-implemented method based on input and commands received from remote computers through the internet. The central computer may effectively serve as a server and the remote computers may serve as client computers such that the server-client relationship is established, and the client computers issue queries or receive output from the server over a network.

The input/output interfaces may include a graphical user interface (GUI) which may be used in conjunction with the computer-executable code and electronic databases. The graphical user interface may allow a user to perform these tasks through the use of text fields, check boxes, pull-downs, command buttons, and the like. A skilled artisan will appreciate how such graphical features may be implemented for performing the tasks of this disclosure. The user interface may optionally be accessible through a computer connected to the internet. In one embodiment, the user interface is accessible by typing in an internet address through an industry standard web browser and logging into a web page. The user interface may then be operated through a remote computer (client computer) accessing the web page and transmitting queries or receiving output from a server through a network connection. In embodiments, the GUI provides reporting functions regarding the status of the code and related medical device construction. Reports may include real-time demonstration of the device construction, status of a brace in a pipeline from data collection through design through fabrication completion. The reports may provide quality control data to show that the device has sufficient strength, fit, and meets the specifications outlined by the data inputs relative to the aspects described in Stage 5. The reports may provide a user the ability to approve an output at any stage of the process to proceed to the following stage, present more data, or perform corrective actions, either manually or automatically by activating a secondary code.

All or part of the product may incorporate aspects of artificial intelligence or machine learning to continually improve the quality of outputs at each stage of the process. In aspects, the machine learning component may optimize the speed and reduce the required computing power of the program by performing any function of Stages 2-5 and optimizing the process (e.g. translations and rotations) to achieve a more direct end result. For example, the algorithm may limit the degrees of motion or minimize the number of translations of a strap slot or other component that is being automatically placed on the device base frame. It may optimize to apply select modifications (e.g. translation and rotation) to groups of components or features in parallel. The machine learning program may optimize to improve device fitment based on data collected following device use by the customer, which may be submitted via a survey or feedback form. Data may be collected via sensors in the device which are uploaded continuously via wifi or bluetooth to a database, or are uploaded at intervals in batches manually or automatically (e.g. an export from the device memory). For example, pressure sensor readings within the brace frame may lead to an automated adjustment of the curvature of the device at a given location based on the average anticipated interaction with the user's anatomy. The machine learning algorithm may analyze this data in combination with initial input data defined in Stage 1 to further optimize design of one subset of devices or all devices within the software's library.

The invention includes devices fabricated using the disclosed method, software system, product, or computer system detailed above.

The present invention has been described with reference to particular embodiments having various features. It will be apparent to those skilled in the art that various modifications and variations can be made in the practice of the present invention without departing from the scope of the invention. One skilled in the art will recognize that these features may be used singularly or in any combination based on the requirements and specifications of a given application or design. Embodiments comprising various features may also consist of or consist essentially of those various features. Other embodiments of the invention will be apparent to those skilled in the art from consideration of the specification and practice of the invention. The description of the invention provided is merely exemplary in nature and, thus, variations that do not depart from the essence of the invention are intended to be within the scope of the invention.

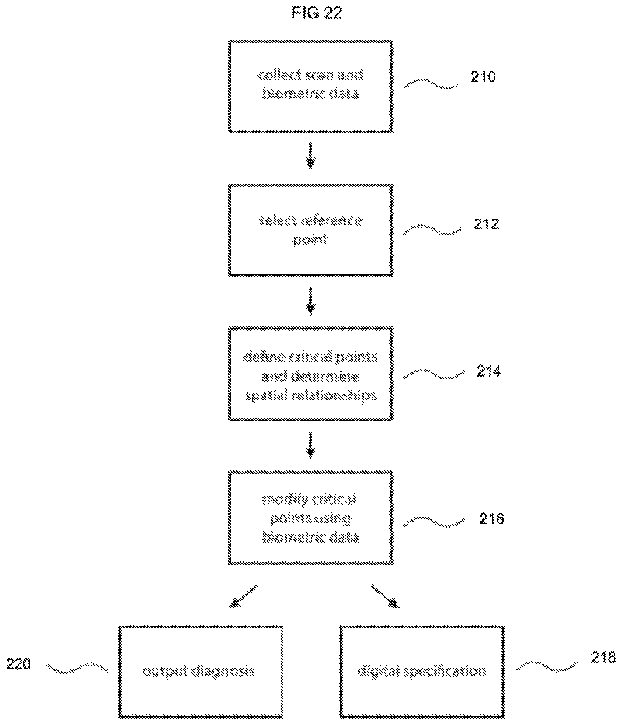

In embodiments, the invention provides for a design and manufacturing method starting, in aspects, from a biological body study until fabrication. An example of workflow is illustrated as a flowchart in FIG. 22. However, the stages may be reordered, skipped, or modified depending on the application.

While knee braces and specific diagnostic applications are used by way of example to describe embodiments of the invention, one skilled in the art would understand that the following descriptions could be applied to any orthotic, prosthetic, wearable device, or user interface (e.g., a handle, vehicle seat, controller, etc.). Furthermore, the current invention as described herein could not only be applied to knee braces, but could also be applied to braces for neck, shoulder, hip, wrist, handle, ankle, elbow, and foot applications, by way of example only. Moreover, the current invention could be applied to wearable technologies and accessories including custom performance wear, watches, helmets, military gear, and footwear, by way of example only. Additionally, embodiments are described for the application of the invention for medical diagnostic approaches, and can be applied to any such diagnostic application where changes in morphology (e.g., swelling or contraction) are measured, by way of example only.

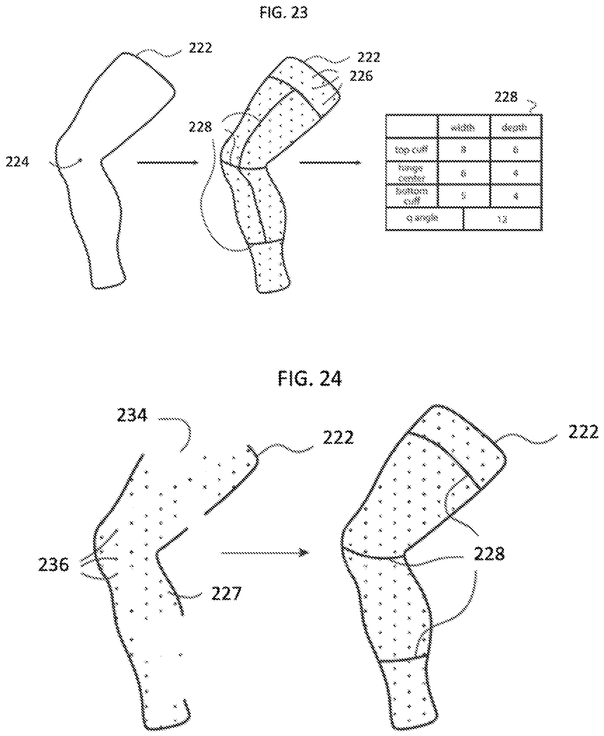

Examples of embodiments of the current invention, including stages and sub-stages, are described below and illustrated in FIG. 22 and FIG. 23.

### Stage A (210): 3D Digital Scanning and Additional Data Collection

For a desired part or parts of the body part / joint/ limb for which the brace, device, wearable or user interface is being designed, data is collected (210), which is then processed to render a 3D digital scan, model, or object (222) (referred to at times herein as object). In some embodiments, this model or scan can be generated directly from the body part / joint/ limb by taking a 3D scan (210) on a device such as a phone, depth camera, tablet computer, or other computer device. The 3D digital model or scan (222) represents a surface to which a brace or device can be relatively fit or contoured. In other embodiments, the 3D digital model or scan (222) can be generated indirectly, for example making a cast of the body part / joint/ limb and then scanning the cast or a mold from the cast. The 3D digital model or scan (222) can be generated based on data representing at least four points in space. Optionally, additional measurements or references can be used to scale, orient, or modify the 3D digital model or scan. In another embodiment, a 3D digital model or scan (222) could be generated from a formula, algorithm, or rendering of a "standard" body part / joint/ limb by scaling, orienting, or modifying the "standard" digital object with external measurements or inputs. For example, circumference measurements of the thigh and calf taken manually by a physician can be input (210) into a system, which will render a 3D digital leg (222) based on a formula of prior measurement data (210) in combination with or independently from 3D model or scan data (210) or 2D pictures.

In some embodiments, internal body/anatomical components (e.g., bones, tendons, cartilage) can be digitally rendered or captured using the 3D scan (210) alone or in addition to other technologies, such as ultrasound, magnetic resonance imaging, x-ray, or scan, which can be further used to modify the 3D digital model or scan. In aspects, internal body/anatomical components can be digitally rendered as a function of the 3D scan data (210), measurements (228), or in reference to the 3D digital model or scan (222). These internal body/ anatomical components can be rendered or captured using the 3D scan (210) alone or in addition to other technologies, such as ultrasound, magnetic resonance imaging, x-ray, or scan.

In some embodiments, the 3D digital model or scan (222) can be further modified by inputs captured through a range of motion to determine the envelope of motion of a joint (e.g., the knee from full flexion through full extension or an ankle in multiple axes of rotation).

Additional biometric, physiological or user preference data (210) can be collected and input into the system manually or automatically (e.g., through data collection via sensor technology). Such data (210) can include quantitative data from a prescribing doctor such as the Q angle of the knee, required varus or valgus correction, required range of motion limitation or augmentation, required support or assistance (e.g., unloading that can be provided by a device that generates force in one or multiple directions). Alternatively, such quantitative data (210) can be determined or measured from the 3D model or scan (210) (e.g., determining the Q angle by identifying the tibial tubercle, the patella, and the quadriceps from the scan). Such data (210) can include patient biometric data (210) or user data such as BMI, age, gender, height, weight, current activity level, desired activity level, and mechanical or bioelectric impedance or elasticity of a part of the body. Such data (210) can include patient qualitative data such as degree of patient pain, location of pain, pain during movement or different activities, pain throughout a range of motion, desired device use, user activity, or lifestyle information.

Further data can be indirectly generated based on initial data inputs (210). For example, the degree of elasticity/mechanical impedance of the thigh, calf or other body part / joint/ limb can be extrapolated from BMI and/or age based on previously defined functions. This would allow for automated design of a device that has dimensions (228) or mechanical properties to provide more compression or a tighter fit in the thigh region, allowing for a better fit, less sliding, and improved performance. This would apply to other body parts, limbs, and joints, as well.

Radiographic data can be included to modify or augment the 3D digital model or scan (222) or other elements of the process to, in aspects, instruct on device shape for corrective purposes such as to restore healthier joint geometry or unload a region of the joint. In some embodiments, data collection (210) can include individually or combinations of Magnetic Resonance Imaging (MRI), Computed Tomography (CT) scan, Position Emission Tomography (PET) scan, X-ray, fluoroscopy or ultrasound data. Such data (210) can reveal cartilage degeneration or osteoarthritis in a joint, inflammation, and/or deformity or damage of bone, cartilage, muscle, tendon, ligament, nerves, skin/epithelium or other tissue, that would automatically or semi-automatically inform device design to correct joint geometry, prevent undesired or unnatural motion, enhance desired motion, or bias the movement of a joint to reduce user pain, for example during gait or a specific activity.

Body parts and objects can be represented as a mesh or other digital file format, a matrix of points (226), a series of splines or a stack of 2D geometries among other representations to form a 3D digital model or scan (222). As shown in FIG. 24, the complete set of data points can comprise the 3D point-cloud data set.

The data is either transferred to a larger database (e.g., external hard drive, server, or the cloud) or used/stored on the capturing device. During the transferring of files, the data may or may not be converted into a new file format. The file can be converted to or from the following formats, however, this list is non-exhaustive: ply, stl, obj, and usdz file extensions. For example, a 3D scan collected on a smartphone app can be stored in a cloud database as a usdz file, which can be further converted to an obj file that becomes the reference model, scan, or object for design in Stage B (212). Optionally, the data can be cleaned to remove extraneous, unwanted, or unneeded features. Methods of data compression can be applied for speed of data access and maintenance of large data sets.

### Stage B (212): Selection of Reference Points (224)

From the 3D point-cloud data set or corresponding digital file format (stl), critical data points (242) required for the manufacture of a selected custom device, defined as "critical points" (242) can be parameterized in order to collect desired measurements (228). In order to generate a clean and usable set of critical points (242) for the application, one or more reference points (224) are to be selected. A reference point (224) is a user or system selected point within the 3D point-cloud data set that represents a morphological feature, for example, around which measurements (228) can be determined, and is used to orient and calibrate additional critical points (242) within the 3D point-cloud data set.

Depending on the brace device to be manufactured or general application, the requirements for the designation of reference points (224) and related critical points (242) are contained within the software. To use an example, to output the digital specifications 2(18) and measurements (228) for a custom knee brace, type 1, model 3, critical points (242) corresponding to the circumference of the knee center, circumference of the thigh 15.24 cm (8 inches) above the knee center, and circumference of the calf 20.32 cm (6 inches) below the knee center are required. In order to properly orient the critical points (242) in space, there needs to be a reference feature, for example, a selected reference point (224). The specification in this example may require three reference points (224) to be selected; one at the center of the patella, one at the center of the thigh, and one at the center of the calf. Alternatively, a reference point corresponding to the center of the patella, and an additional reference point (224) corresponding to the center of the medial condyle may need to be selected. Reference points (224) may be selected manually by the user to identify key morphological features. Secondary reference points (224) can be selected automatically based on known relations within the 3D point-cloud data and the initial selected reference point (224). For example, the position of reference points (224) on the medial and lateral condyles of the knee may be able to be identified automatically relative to an initial reference point (224) at the knee center. This can be done through established algorithms defining the surface morphology of the knee, extrapolation/ interpolation of geometric relationships between local data points within the 3D point-cloud data set, or extrapolation/ interpolation of geometric relationships from a database of other similar body part / joint/ limb scans, including the use of machine learning applications. The reference points (224) act to properly orient coordinate systems and further define the position of critical points (242) within that system, between which measurements (228) can be taken. Reference points (224) support the generation of a set of critical points (242) with defined position(s).

### Stage C (214): Defining Critical Points (242) and Spatial Relationships

In aspects, critical points (242) represent data points within the 3D point-cloud data set whose position must be known for the effective specification of a device manufacturing protocol. In other embodiments, critical points (242) may be unknown if the 3D point-cloud data set is incomplete (234), in which case critical points (242) can be extrapolated or interpolated from the existing 3D point-cloud data. A mesh may consist of a series of critical points (242) that represent the 3D configuration of the point-cloud data set (227). Alternatively, they can represent the path of the device frame to be manufactured. The critical points (242) are oriented relative to a world space coordinate system (e.g., a cylindrical system applied to a leg). Their relationship provides reference frames to extract physical (condyle, patella) and dimensional (mm length, angle, etc.) information from.

Physical features are extracted with image processing, machine learning, convolutions, and a combination of measurements as described herein.

Measurements (228) are taken from specific reference frames, e.g., (240), to the critical points (242) located in 3D space. In aspects, the reference position and direction vectors required in the designation of critical points (242) are predetermined based on engineering design considerations. As the reference frames move through the domain, measurements (228) are taken to construct a new field of data. The data points are then connected with bezier curves in a way that represents an abstracted version of the mesh. The data points (226) and bezier curves are used to determine various lengths (228), circumferences (228), and relationships (228) that drive engineering design features.

### Stage D (216): Modification of Critical Points (242) based on Biometric Information (210)

At any stage of the process, individual data points (226), groupings of data points, surfaces, bezier curves, or other splines containing data sets can be transformed. Transformations including translations, rotations, mirroring, and/or isotropic or anisotropic scaling are performed to move a reference object into a new location. Such transformations may take into account biometric data (210). For example, a scan for a patient of a higher BMI may move data points closer together to represent intentional compression of leg tissue performed by the fabricated device that will improve brace fit and/or function. Alternatively, the transformations can alter the Q angle of the leg from the current to an optimal position to unload a compartment of the joint or alter the gait of the individual, and incorporate that information into the device fabrication instructions (218). These transformations can be applied to data within the 3D point-cloud data set (227), to the reference point(s) (224), the critical point(s) (242), or a digitally rendered model of the device to be fabricated, for example.

In other embodiments, qualitative and quantitative data from physicians (e.g., medical provider recommendation or patient surveys input in Stage A (or at any point in the overall process)) may cause modifications to the device to improve the desired physiological function. For example, a patient's reported pain on the medial side of the knee with a recommended Q angle correction of 3° would adjust the brace frame for a correction to unload the medial compartment of the knee, and would be reflected in the digital specifications or digital model (e.g., a virtual custom device or virtual custom brace) of the brace or device to be manufactured.

In other embodiments, radiographic information may lead to automated adjustment of the critical points (242). For example, the identification of arthritis in the medial compartment of the knee can trigger a similar brace frame adjustment as described in the previous example. It is worth noting that in the two adjustment examples described above, the specification and resulting geometry of the brace frame is purposely altered such that it no longer unintelligently conforms to the 3D scan of the body part / joint/ limb, rather it now intelligently applies a sideways force to the knee joint to unload it when the patient tightens the straps, for example.

In other embodiments, patient data, including biometric data (210), can lead to automated adjustment of the device. For example, for an individual with an above average BMI, the top brace frame can be adjusted for a tighter fit and greater compression of the adipose tissue in the thigh. It is intended that by taking into account the amount of adipose tissue as indicated by the BMI value, the resulting fit would improve the function of the brace (by preventing migration) better than a one-to-one fit of the frame to the 3D image of the upper leg.

In other embodiments, scan data (210) showing envelope of motion for a joint can adjust design to improve or optimize the axis of rotation of a device throughout a range of motion. The adjustment can limit or augment movement of the joint or body point in a given direction based on a gait pattern in combination with or independently from data from the physician or radiographic data. For example, the length of a slot in a hinge through which a bolt travels during brace flexion and extension can be limited to reduce the travel of the bolt, thereby limiting the user to a range of motion of 20° to 125° of flexion.

In embodiments involving diagnostic applications, additional biometric or historical scan data (210) may be required. The program may consider past measurements (228) for the individual to indicate if dimensions have increased, decreased, or changed beyond an expected rate. Historical data from individuals or from a database of other individuals can be referenced. For example, a risk of hydrocephalus in babies is initially identified by manually measuring the head of the child with a measuring tape and comparing that value to an expected value or range of values based on the child's growth stage. To apply the disclosed invention for this diagnostic application, a professional can scan the child's head and input additional data such as age (or date of birth) and weight. Then, by comparing to a database of expected measurements, including but not limited to circumference, volume, or sphericity, based on the child's measurement history and other children with a similar biometric or physiological profile, it can be determined whether the child presents a normal or abnormal condition.

Application of the technology for a similar application could determine sphericity of the skull to indicate whether the child should be prescribed a cranial orthosis for helmet molding therapy to correct the shape of the child's head.

Another application of the technology involves diagnosis of thrombosis, for example deep vein thrombosis in the leg. An increase in the volume or circumference of the leg as determined by a scan in combination with the methods described herein, would signal presence of deep vein thrombosis which causes swelling of the affected limb. The data could be compared to the user's other leg, or against a database of legs where an expected outcome is presented based on the user's biometric or physiological profile.

The benefit of using the described invention as opposed to current methods for each of these diagnostic applications is that the method is more precise and eliminates or decreases user error to improve accuracy of diagnosis. Additionally, diagnosis can be presented earlier due to the precision of the method, compared to current methods, which yields significant benefits in terms of patient safety.

### Presentation of Digital Specification for Manufacturing or Diagnostic Output

Depending on the application, specific measurements (228) will be presented to the user on a user interface. The user can be a patient, a wearer of the brace or device, a medical provider, a manufacturer, or a medical professional of any kind. The information can present the measurements (228) of the body or body part / joint/ limb at different points in general, for example the circumference of the knee center, the thigh 6" above knee center, and the calf 6" below the knee center. The information can be presented as raw measurements corresponding to different dimensions of the device to be fabricated, for example the width and depth of the top and bottom cuffs of a knee brace (218). Measurements (228) can be presented along with specifications (218) for fabrication (218), which contain the measurements (228) at the relevant point of the manufacturing process. In one example, the specifications (218) can provide for the modification of a component to fit a specific measurement, for example for the thermo-forming of an upper brace frame cuff to a width of 12" and depth of 3". Alternatively, the specifications (218) can provide for the specific device components to be selected in manufacture. By way of example, a strap of 12" length may be selected by the fabricator based on the collected measurement (228) and resulting digital specifications (218). It should be noted that the component to be selected may not directly match the precise measurement collected. For example, a strap of 12" in length is selected for an individual with a calf circumference between 9" to 13" whereas a strap of 16" in length is selected for an individual with a calf circumference between 13" to 16" in diameter. Specific components may be included or not included depending on the collected measurement(s) (228) and resulting specifications (218). For example the digital specification (218) for a knee brace for an individual with a thigh circumference above 22" and BMI above 30 may include an additional strap component to prevent migration of the device.

Wherein the abovementioned inch units are converted in the following manner: 3"= 7.62 cm, 6"=15.24 cm, 9"= 2.86 cm, 12"=30.48 cm, 13"=33.02 cm, 16"=40.64 cm and 22"=55.88 cm.

The measurements (228) or specifications (218) can be presented on variable user interfaces, including but not limited to mobile devices, computers, print-outs, audio or hosted on a website. The measurements (228) and instructions (218) can accompany a digital representation of the device to be fabricated, which can be presented on the user interface, which can include virtual or augmented reality.

A standard protocol for a specific device and fabrication process is designed and supported by unique software. In embodiments, the software can apply decision trees, either selected by the user at stages of the process or automated depending on the type of scan data (210) collected. While Stages A through C described above can be identical for different applications, Stage D (216) can vary to specify which set of measurements (228) are required. Alternatively, specifications that drive the underlying code for stages B through D can change based on the application. In Stage A (210), selection of the reference point's (224) location and also the number of reference points (224) can be dictated by the specific device, body part/ joint/ limb, fabrication process, or desired fabrication specifications (218). For example, automated measurement for a knee brace may only require selection of a reference point (224) representing the center of the patella. Alternatively, automated measurement of an ankle-foot complex for an ankle-foot orthosis may require reference points (224) representing the heel, toes, and medial center of the ankle joint.

The same differences in application may drive the underlying software operations for Stage C (214). The density of the critical points (242) or mesh, number of data points presented and interpolation or extrapolation of additional data points may vary depending on the application to achieve a specific degree of precision or outcome. The processes and algorithms applied to generate the mesh of parameterized critical points (242), as described in Stage C, may determine the formation of that mesh.

Lastly, in aspects, measurement operations performed by the software will be determined by the application, and based on user information. For example, the bezier curves, splines, lines or planes populated or selected within the mesh for measurement may be dependent on the individual, device, or application. For example, additional curves and corresponding measurements of those curves can be presented if the height of the individual is above 182,88 cm (6 feet) tall in order to provide additional information for a longer brace frame.

In embodiments, the user may preselect the type of device, fabrication process, and user biometrics (210) to determine the path that is taken for stages B-D within the software (e.g., an automated decision tree). Alternatively, artificial intelligence may be applied to automatically identify the desired device based on the type of body part / joint/ limb scanned in Stage A (210) (e.g., the protocol for an ankle brace will be automatically run if an ankle is scanned while the protocol for a shoulder brace will be run if a shoulder is scanned). The software can be able to determine the selection by comparing the scan to a database of existing scans that have associated body part / joint/ limb or orthotic device labels.

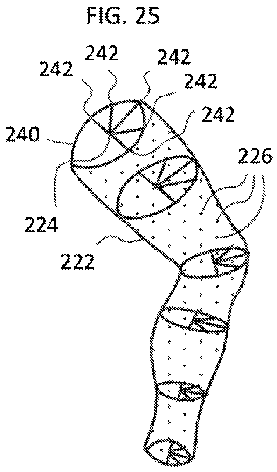

The following stages may be done in any particular order, during any of the above described stages, or between any of the above described stages. In embodiments, some or all of Stages B through D may occur in a loop and incorporate the steps described herein in order to yield a target digital specification (218) or a digital specification (218) up to the quality standards of the device to be manufactured. An example is described below and displayed in Fig. 25. The data file from Stage A (210) may be cleaned to remove irregularities, soften extrema, connect surfaces, or modify locations. Cleansing may take the form of removing, adding or joining points, lines, planes, or surfaces. Data compression may also occur to improve overall efficiency, reduce operation time, and minimize required computing power.

### Interpolation, Extrapolation of Data and Scan Repair

In some instances, the 3D point-cloud data set (227) may be incomplete or insufficient to yield the data required to determine the measurements (228) needed. In some cases, the software can identify that incomplete data (234) has been collected and the user of the scanning software may be presented with an error message indicating to rescan the body part / joint/ limb. Alternatively, the disclosed invention provides a method and software through which the captured 3D point-cloud data set (227) can be expanded based on algorithms (e.g., as determined by a machine learning application of multiple scans lacking this data and presented with the actual data, or e.g., as determined and experimentally validated based on equations defining the morphology of a wearer's leg using inputs such as height, weight, BMI, and existing scan data). The same techniques may be applied to repair gaps in the model or scan (234) or populate or complete expected features within the scan, for example a portion of the patella. In other instances, user error may yield incomplete (234) 3D point-cloud data sets (227). Similarly, bumps or ridges formed due to scanning technology or user error can be identified and eliminated to yield a usable and accurate data set (222). Such discontinuities can be identified based on a divergence from "expected" or "regular" morphology, which is either defined within the software, determined based on local geometric relationships within the existing 3D point-cloud data set (227), or determined based on machine learning applications using a database of similar scan data. In such cases, the scan is reworked to provide a 3D point-cloud data set (227), reference points (224), or critical points (242) that can be used by the described application.

Lastly, a quality control element of the automated measurement may be applied to the data collected or a digital device model (222). Alternatively, instructions on a quality control procedure or expected quality outcomes for the device to be fabricated may be generated. This element may include checking bounds of mass, the bounds of size, the curvature of the body, continuity of bodies within the device, and bounds of clearance values to ensure the device meets specifications (218) needed for manufacturing. Other quality control automations such as finite element analysis for mechanical testing ensure that the device meets functional needs.

In aspects, quality control measurements, dimensions, or criteria can optionally be generated from the automation. For example, the minimal path length of the inner surface of the upper portion of a knee brace may be calculated to ensure a minimum structural integrity while also achieving the required fit based on the measurements (228) and additional biometric data (210). These measurements could be used during a physical check of the post-manufactured device or to assist an artisan in the hand crafting or modification of a part. In yet another aspect, the measurements, dimensions, or criteria generated from the automation could be used for non-manufacturing purposes, such as diagnostics (220). For example, an algorithm could determine the degree of swelling of an elbow joint or quantify the size of a protuberance from the automation file.

In the case that the measurements (228) are applied to a digital device model (222), the model can then be converted into a physical model of the device using either additive or subtractive manufacturing techniques, or combinations thereof.

With the current measurement process, which enables autonomous vertical integration of manufacturing herein, one of several improvements is to the time required to produce the device. According to the present invention, the consumer may receive the necessary device on a shortened time frame. Due to the current invention, devices can now be produced and purchased at a lower cost. This allows for a larger market to interact with the object - improving the quality of life of a larger populus.

Application of advanced measurement, and incorporation of software to reduce human error while simplifying the measurement procedure yield a superior device with improved outcomes at scale, compared to the average product that could be produced through existing methods. The ability to decide the form based on tangibles creates a superior device and decreases or eliminates the possibility of human error during device creation. Superiority comes in the form of improved fitment, increased comfort, greater ultimate strength, better orthotic function, and a larger resistance to shearing forces.

Lastly, the automation allows for a consistent device. Despite being custom, a wearer will be able to accurately predict the interaction the device has with his or her body and have confidence in the fit and function.

In application of the invention, by way of a non-limiting example, a patient with multicompartmental knee OA, including patellofemoral and medial compartment OA, would visit a doctor/medical provider after experiencing knee pain during activity. The patient may also consult a doctor remotely using telehealth. The doctor would identify indications of knee OA, including reduced stability, user pain during specific activities such as climbing stairs, and potential joint deformity. At this stage, a custom brace may be prescribed. The doctor, another health professional, a technician, or the user may scan the joint using software on a phone, tablet, or other computer or digital device. The scan data (210) would then be uploaded to a cloud-based server. Data may be cleaned, processed, or converted into a different file type on the device used to take the scan or after being transferred to the server. The prescriber may also provide supporting radiographic data, such as an MRI, through the health network's user interface, which may then be sent to the same or a different cloud-based server. Data from the same server or different servers within the network may be paired by a unique identifier, such as a patient or job ID number. Quality analysis of the data may be performed either on the device or user interface where the data is collected or in the server on which the data is stored. The user or doctor may receive error messages indicating that data needs to be recaptured if it does not pass quality standards.

Processed data will then be transferred to a computer system on which the software comprising the code for operations from Stages B through D are performed (as described above). An operator may oversee the process as performed by the software. The software may present them with reports, in real time or at different stages of the process to assess the accuracy and progress of the process. A digital specification is thereby generated combining elements from scan or model, the supporting radiographic data, and such elements as prescribed by the doctor/medical provider from which a manufacturing measurement system can be derived. The measurement system may be integrated with the fabrication system, which controls 3D printing output, through an API to automatically queue and print the digitally represented device. The final device may be fabricated as one continuous part from one or more materials. Alternatively, additional components may be added during an assembly process to yield a final device. The entire process, from data collection (210) to delivery of a final, custom fit device to fulfill the user need may be completed, in aspects, within 12 hours based on the current, automated system as described.

Embodiments of the invention also include a computer readable medium comprising one or more computer files comprising a set of computer-executable instructions for performing one or more of the calculations, stages, processes and operations described and/or depicted herein. In exemplary embodiments, the files may be stored contiguously or non-contiguously on the computer-readable medium. Embodiments may include a computer program product comprising the computer files, either in the form of the computer-readable medium comprising the computer files and, optionally, made available to a consumer through packaging, or alternatively made available to a consumer through electronic distribution. As used in the context of this specification, a "computer-readable medium" is a non-transitory computer-readable medium and includes any kind of computer memory such as floppy disks, conventional hard disks, CD-ROM, Flash ROM, non-volatile ROM, electrically erasable programmable read-only memory (EEPROM), and RAM. In exemplary embodiments, the computer readable medium has a set of instructions stored thereon which, when executed by a processor, cause the processor to perform tasks, based on data stored in the electronic database or memory described herein. The processor may implement this process through any of the procedures discussed in this disclosure or through any equivalent procedure.

In other embodiments of the invention, files comprising the set of computer-executable instructions may be stored in computer-readable memory on a single computer or distributed across multiple computers. A skilled artisan will further appreciate, in light of this disclosure, how the invention can be implemented, in addition to software, using hardware or firmware. As such, as used herein, the operations of the invention can be implemented in a system comprising a combination of software, hardware, or firmware.

Embodiments of this disclosure include one or more computers or devices loaded with a set of the computer-executable instructions described herein. The computers or devices may be a general purpose computer, a special-purpose computer, or other programmable data processing apparatus to produce a particular machine, such that the one or more computers or devices are instructed and configured to carry out the calculations, processes, stages, operations, algorithms, statistical methods, formulas, or computational routines of this disclosure. The computer or device performing the specified calculations, processes, stages, operations, algorithms, statistical methods, formulas, or computational routines of this disclosure may comprise at least one processing element such as a central processing unit (i.e., processor) and a form of computer-readable memory which may include random-access memory (RAM) or read-only memory (ROM). The computer-executable instructions can be embedded in computer hardware or stored in the computer-readable memory such that the computer or device may be directed to perform one or more of the calculations, stages, processes and operations depicted and/or described herein.

Additional embodiments of this disclosure comprise a computer system for carrying out the computer-implemented method of this disclosure. The computer system may comprise a processor for executing the computer-executable instructions, one or more electronic databases containing the data or information described herein, an input/output interface or user interface, and a set of instructions (e.g., software) for carrying out the method. The computer system can include a stand-alone computer, such as a desktop computer, a portable computer, such as a tablet, laptop, PDA, or smartphone, or a set of computers connected through a network including a client-server configuration and one or more database servers. The network may use any suitable network protocol, including IP, UDP, or ICMP, and may be any suitable wired or wireless network including any local area network, wide area network, Internet network, telecommunications network, Wi-Fi enabled network, or Bluetooth enabled network. In one embodiment, the computer system comprises a central computer connected to the internet that has the computer-executable instructions stored in memory that is operably connected to an internal electronic database. The central computer may perform the computer-implemented method based on input and commands received from remote computers through the internet. The central computer may effectively serve as a server and the remote computers may serve as client computers such that the server-client relationship is established, and the client computers issue queries or receive output from the server over a network.

The input/output interfaces may include a graphical user interface (GUI) which may be used in conjunction with the computer-executable code and electronic databases. The graphical user interface may allow a user to perform these tasks through the use of text fields, check boxes, pull-downs, command buttons, and the like. A skilled artisan will appreciate how such graphical features may be implemented for performing the tasks of this disclosure. The user interface may optionally be accessible through a computer connected to the internet. In one embodiment, the user interface is accessible by typing in an internet address through an industry standard web browser and logging into a web page. The user interface may then be operated through a remote computer (client computer) accessing the web page and transmitting queries or receiving output from a server through a network connection. In embodiments, the GUI provides reporting functions regarding the status of the code and related medical device construction. Reports may include real-time demonstration of the device construction, status of a brace in a pipeline from data collection through design and/or through fabrication completion. The reports may provide quality control data to show that the device has sufficient strength, fit, and/or meets the specifications (218) outlined by the data inputs. The reports may provide a user the ability to approve an output at any stage of the process to proceed to the following stage, present more data, or perform corrective actions, either manually or automatically by activating, for example, a secondary code.

All or part of the product may incorporate aspects of artificial intelligence or machine learning to continually improve the quality of outputs at each stage of the process. In aspects, the machine learning component may optimize the speed and reduce the required computing power of the program by performing any function of Stages B-D and optimizing the process to achieve a superior functional outcome or more accurate diagnoses. The machine learning program may optimize to improve device fitment based on data collected following device use by the customer, which may be submitted via a survey or feedback form. Data may be collected via sensors in the device which are uploaded continuously via wifi or bluetooth to a database, or are uploaded at intervals in batches manually or automatically (e.g., an export from the device memory). For example, pressure sensor readings within the brace frame may lead to an automated adjustment of the curvature of the device at a given location based on the average anticipated interaction with the user's anatomy. The machine learning algorithm may analyze this data in combination with initial input data defined in Stage A (210) to further optimize design of one subset of devices or all devices within the software's library.

The invention includes devices fabricated using the disclosed method, software system, product, or computer system detailed above.

It is also apparent that the skilled artisan could easily modify the dimensions, materials, number and type of tensioning elements, and so forth to achieve an equivalent level of pain relief as the embodiments disclosed herein.

Although various features of the invention may be described in the context of a single embodiment, the features may also be provided separately or in any suitable combination. Conversely, although the invention may be described herein in the context of separate embodiments for clarity, the invention may also be implemented in a single embodiment.

As used herein, the term "about" refers to plus or minus 5 units (e.g., percentage) of the stated value.

Reference in the specification to "some embodiments", "an embodiment", "one embodiment" or "other embodiments" means that a particular feature, structure, or characteristic described in connection with the embodiments is included in at least some embodiments, but not necessarily all embodiments, of the inventions.

As used herein, the term "substantial" and "substantially" refers to what is easily recognizable to one of ordinary skill in the art.

It is to be understood that the phraseology and terminology employed herein is not to be construed as limiting and are for descriptive purpose only.

It is to be understood that the details set forth herein do not construe a limitation to an application of the invention.

Furthermore, it is to be understood that the invention can be carried out or practiced in various ways and that the invention can be implemented in embodiments other than the ones outlined in the description above.

It is to be understood that the terms "including", "comprising", "consisting" and grammatical variants thereof do not preclude the addition of one or more components, features, steps, or integers or groups thereof and that the terms are to be construed as specifying components, features, steps or integers.

## Claims

1. A joint brace (11) comprising:
a support comprising an upper portion (1) and a lower portion (2);
**characterised by** a distracting hinge (22, 23) connected to the upper portion and the lower portion, wherein the distracting hinge comprises teethed gears (24, 25, 26, 27), curved adjacent surfaces, or a combination thereof, which comprise variable radii, the radii varying with degree of flexion and providing joint distraction upon articulating or moving near or against each other.

2. The joint brace of claim 1, further comprising a tensioning element (9), wherein the tensioning element generates tension as the tensioning element is drawn over the distracting hinge between the upper portion and the lower portion, or the tensioning element is located within either or both the upper portion and the lower portion and attached to wires or lace that are drawn over the distracting hinge.

3. The joint brace of claim 1, wherein the upper portion and the lower portion are connected by a center cap (6), wherein one or more pins connect the center cap to the upper portion and the lower portion, and wherein the center cap comprises slots allowing the upper portion and the lower portion to move apart as the radii of the variable radii increase, move closer together as the radii of the variable radii decrease, or combinations thereof.

4. The joint brace of claim 1, wherein the upper portion and the lower portion are connected by a center cap (6) and one or more pins, and wherein a force is applied directly or indirectly to the one or more pins providing a counter-force between the teethed gears, the curved adjacent surfaces, or a combination thereof, of the distracting hinge.

5. The joint brace of claim 1, wherein the distracting hinge, the upper portion, the lower portion, or combinations thereof, are shaped to a user's joint or limb based on a joint or limb measurement, three-dimensional scan of the user's joint or limb, or radiographic scan of the user's joint or limb.

6. The joint brace of claim 1, wherein the distracting hinge is curved or adapted to be conformed to a user's anatomy.

7. The joint brace of claim 1, wherein the joint distraction occurs only over part of a range of articulation of the distraction hinge.

8. The joint brace of claim 2, wherein a user can adjust an amount of tension in the tensioning element, an amount of joint distraction, or both using an adjustment mechanism.

9. The joint brace of claim 2, wherein the tensioning element is drawn over the at least one pivoting hinge, or wherein the tensioning element is integrated within the upper portion, the lower portion, or both, wherein the tensioning element is connected to a lace or wire drawn over the one or more pivoting hinge, or combinations thereof.

10. A computer-implemented method for automating the design and manufacture of a joint brace according to claim **1,** the method comprising:
providing a digital depth camera capable of three-dimensional (3D) scanning;
obtaining a 3D surface scan using the depth camera;
processing compiled scan data;
defining geometric constraints based on the scan data;
digitally fitting a virtual 3D pre-designed base device to the obtained 3D surface scan;
adapting the virtual 3D pre-designed base device to a virtual custom device based on the obtained 3D surface scan and the defined geometric constraints; and
manufacturing the joint brace or orthotic device based on the virtual custom device.

11. The joint brace of claim 1, wherein the upper portion and the lower portion of the joint brace are connected by at least one lace, cable or wire.

## Patentansprüche

1. Gelenkorthese (11), umfassend:
eine Stütze, die einen oberen Abschnitt (1) und einen unteren Abschnitt (2) umfasst;
**gekennzeichnet durch** ein distrahierendes Scharnier (22, 23), das mit dem oberen Abschnitt und dem unteren Abschnitt verbunden ist, wobei das distrahierende Scharnier gezahnte Zahnräder (24, 25, 26, 27), gekrümmte benachbarte Oberflächen oder eine Kombination davon umfasst, die variable Radien umfassen, wobei die Radien mit dem Grad der Flexion variieren und eine Gelenkdistraktion beim Artikulieren oder Bewegen nahe oder gegeneinander bereitstellen.

2. Gelenkorthese nach Anspruch 1, ferner umfassend ein Spannelement (9), wobei das Spannelement Spannung erzeugt, wenn das Spannelement über das distrahierende Scharnier zwischen dem oberen Abschnitt und dem unteren Abschnitt gezogen wird, oder das Spannelement innerhalb des oberen Abschnitts und/oder des unteren Abschnitts angeordnet und an Drähten oder Schnüren befestigt ist, die über das distrahierende Scharnier gezogen werden.

3. Die Gelenkorthese nach Anspruch 1, wobei der obere Abschnitt und der untere Abschnitt durch eine Mittelkappe (6) verbunden sind, wobei ein oder mehrere Stifte die Mittelkappe mit dem oberen Abschnitt und dem unteren Abschnitt verbinden, und wobei die Mittelkappe Schlitze umfasst, die es dem oberen Abschnitt und dem unteren Abschnitt ermöglichen, sich voneinander zu entfernen, wenn sich die Radien der variablen Radien vergrößern, sich einander zu nähern, wenn sich die Radien der variablen Radien verkleinern, oder Kombinationen davon.

4. Gelenkorthese nach Anspruch 1, wobei der obere Abschnitt und der untere Abschnitt durch eine Mittelkappe (6) und einen oder mehrere Stifte verbunden sind, und wobei eine Kraft direkt oder indirekt auf den einen oder die mehreren Stifte aufgebracht wird, die eine Gegenkraft zwischen den Zahnrädern, den gekrümmten benachbarten Oberflächen oder einer Kombination davon des distrahierenden Scharniers bereitstellt.

5. Gelenkorthese nach Anspruch 1, wobei das distrahierende Scharnier, der obere Abschnitt, der untere Abschnitt oder Kombinationen davon an das Gelenk oder die Extremität eines Benutzers basierend auf einer Gelenk- oder Extremitätenmessung, einem dreidimensionalen Scan des Gelenks oder der Extremität des Benutzers oder einem radiographischen Scan des Gelenks oder der Extremität des Benutzers angeformt sind.

6. Gelenkorthese nach Anspruch 1, wobei das distrahierende Scharnier gekrümmt ist oder dazu ausgelegt ist, an die Anatomie eines Benutzers angepasst zu werden.

7. Gelenkorthese nach Anspruch 1, wobei die Gelenkdistraktion nur über einen Teil eines Artikulationsbereichs des Distraktionsscharniers auftritt.

8. Gelenkorthese nach Anspruch 2, wobei ein Benutzer eine Spannungsmenge in dem Spannelement, eine Menge der Gelenkdistraktion oder beides unter Verwendung eines Stellmechanismus einstellen kann.

9. Gelenkorthese nach Anspruch 2, wobei das Spannungselement über das mindestens eine Schwenkscharnier gezogen wird, oder wobei das Spannungselement in dem oberen Abschnitt, dem unteren Abschnitt oder beiden integriert ist, wobei das Spannungselement mit einer Schnur oder einem Draht verbunden ist, die/der über das eine oder die mehreren Schwenkscharniere gezogen wird, oder Kombinationen davon.

10. Computerimplementiertes Verfahren zur Automatisierung der Konstruktion und Herstellung einer Gelenkorthese nach Anspruch 1, wobei das Verfahren umfasst:
Bereitstellen einer digitalen Tiefenkamera, die zur dreidimensionalen Abtastung (3D-Abtastung) fähig ist;
Erhalten eines 3D-Oberflächenscans unter Verwendung der Tiefenkamera;
Verarbeiten kompilierter Scandaten;
Definieren geometrischer Beschränkungen basierend auf den Scandaten;
digitales Anpassen einer virtuellen vorgestalteten 3D-Basisvorrichtung an den erhaltenen 3D-Oberflächenscan;
Anpassen der virtuellen vorgestalteten 3D-Basisvorrichtung an eine virtuelle kundenspezifische Vorrichtung basierend auf dem erhaltenen 3D-Oberflächenscan und den definierten geometrischen Beschränkungen; und
Herstellen der Gelenkorthese oder orthopädischen Vorrichtung basierend auf der virtuellen kundenspezifischen Vorrichtung.

11. Gelenkorthese nach Anspruch 1, wobei der obere Abschnitt und der untere Abschnitt der Gelenkorthese durch mindestens eine Schnur, ein Kabel oder einen Draht verbunden sind.

## Revendications

1. Attelle d'articulation (11) comprenant :
un support comprenant une partie supérieure (1) et une partie inférieure (2) ;
**caractérisée par** une charnière de distraction (22, 23) reliée à la partie supérieure et à la partie inférieure, dans laquelle la charnière de distraction comprend des engrenages dentés (24, 25, 26, 27), des surfaces adjacentes incurvées, ou une combinaison de celles-ci, qui comprennent des rayons variables, les rayons variant avec le degré de flexion et fournissant une distraction articulaire lors de l'articulation ou du déplacement à proximité ou contre l'autre.

2. Attelle d'articulation selon la revendication 1, comprenant en outre un élément de tension (9), dans laquelle l'élément de tension génère une tension lorsque l'élément de tension est tiré sur la charnière de distraction entre la partie supérieure et la partie inférieure, ou que l'élément de tension est situé à l'intérieur de la partie supérieure et/ou de la partie inférieure et attaché à des fils ou à des dentelles qui sont tirés sur la charnière de distraction.

3. Attelle d'articulation selon la revendication 1, dans laquelle la partie supérieure et la partie inférieure sont reliées par un capuchon central (6), dans laquelle une ou plusieurs broches relient le capuchon central à la partie supérieure et à la partie inférieure, et dans laquelle le capuchon central comprend des fentes permettant à la partie supérieure et à la partie inférieure de s'écarter à mesure que les rayons des rayons variables augmentent, se rapprochent à mesure que les rayons des rayons variables diminuent, ou des combinaisons de ceux-ci.

4. Attelle d'articulation selon la revendication 1, dans laquelle la partie supérieure et la partie inférieure sont reliées par un capuchon central (6) et une ou plusieurs broches, et dans laquelle une force est appliquée directement ou indirectement à la ou aux goupilles fournissant une contre-force entre les engrenages dentés, les surfaces adjacentes incurvées, ou une combinaison de celles-ci, de la charnière gênante.

5. Attelle d'articulation selon la revendication 1, dans laquelle la charnière de distraction, la partie supérieure, la partie inférieure, ou des combinaisons de celles-ci, sont formées à l'articulation ou au membre d'un utilisateur sur la base d'une mesure d'articulation ou de membre, d'un balayage tridimensionnel de l'articulation ou du membre de l'utilisateur, ou d'un balayage radiographique de l'articulation ou du membre de l'utilisateur.

6. Attelle d'articulation selon la revendication 1, dans laquelle la charnière de distraction est incurvée ou adaptée pour être conforme à l'anatomie d'un utilisateur.

7. Attelle d'articulation selon la revendication 1, dans laquelle la distraction articulaire ne se produit que sur une partie d'une plage d'articulation de la charnière de distraction.

8. Attelle d'articulation selon la revendication 2, dans laquelle un utilisateur peut régler une quantité de tension dans l'élément de tension, une quantité de distraction d'articulation, ou les deux à l'aide d'un mécanisme d'ajustement.

9. Attelle d'articulation selon la revendication 2, dans laquelle l'élément de tension est tiré sur l'au moins une charnière pivotante, ou dans laquelle l'élément de tension est intégré dans la partie supérieure, la partie inférieure, ou les deux, dans laquelle l'élément de tension est relié à un lacet ou à un fil tiré sur la ou les charnières pivotantes, ou des combinaisons de ceux-ci.

10. Procédé mis en œuvre par ordinateur permettant d'automatiser la conception et la fabrication d'une attelle d'articulation selon la revendication 1, le procédé comprenant :
la fourniture d'une caméra de profondeur numérique capable de balayer en trois dimensions (3D) ;
l'obtention d'un balayage de surface 3D à l'aide de la caméra de profondeur;
le traitement des données de balayage compilées ;
la définition de contraintes géométriques en fonction des données de balayage ;
l'adaptation numérique d'un dispositif de base 3D virtuel préconçu au balayage de surface 3D obtenu ;
l'adaptation du dispositif de base préconçu en 3D virtuel à un dispositif personnalisé virtuel sur la base du balayage de surface 3D obtenu et des contraintes géométriques définies ; et
la fabrication de l'attelle d'articulation ou du dispositif orthétique sur la base du dispositif personnalisé virtuel.

11. Attelle d'articulation selon la revendication 1, dans laquelle la partie supérieure et la partie inférieure de l'attelle d'articulation sont reliées par au moins une dentelle, un câble ou un fil.
